# EUROPEAN PATENT SPECIFICATION

(11) **EP 3 223 835 B1**
(45) Date of publication and mention of the grant of the patent: **13.11.2024**
(21) Application number: 15862844.6
(22) Date of filing: 25.11.2015
(51) Int. Cl.: A61K 35/74, A61K 31/70, A61K 31/715, A61P 35/00, C12Q 1/689

(54) **INTESTINAL MICROBIOTA AND GVHD**
DARMFLORA UND TRANSPLANTAT-GEGEN-EMPFÄNGER-REAKTION (GVHD)
MICROBIOTE INTESTINAL ET GVH (RÉACTION DU GREFFON CONTRE L'HÔTE)

(30) Priority: 25.11.2014 US 201462084219 P; 19.01.2015 US 201562105063 P; 04.02.2015 US 201562111949 P
(43) Date of publication of application: 04.10.2017
(73) Proprietor: Memorial Sloan-Kettering Cancer Center, New York, NY 10065 (US)
(72) Inventor: VAN DEN BRINK, Marcel, New York, NY 10065 (US); JENQ, Robert, New York, NY 10022 (US); PAMER, Eric G., Guilford, CT 06437 (US); TAUR, Ying, Hastings on Hudson NY 10706 (US); SHONO, Yusuke, Woodside, NY 11137 (US)
(74) Representative: Barker Brettell LLP
(86) International application number: PCT/US2015/062734
(87) International publication number: WO 2016/086161

(56) References cited:
- WO-A1-2013/016636
- WO-A2-2013/177596
- WO-A2-2014/153194
- US-A1- 2013 115 232
- US-A1- 2014 199 281
- US-A1- 2014 328 803
- US-A1- 2014 341 921
- JENQ ET AL.: "Regulation of intestinal inflammation by microbiota following allogeneic bone marrow transplantation", J EXP MED., vol. 209, 30 April 2012 (2012-04-30), pages 903 - 911, XP055445918
- KAMBOJ ET AL.: "Clostridium difficile infection after allogeneic hematopoietic stem cell transplant: strain diversity and outcomes associated with NAP1/027.", BIOL BLOOD MARROW TRANSPLANT., vol. 20, 25 June 2014 (2014-06-25), pages 1626 - 1633, XP029051625

## Description

### Statement Regarding Federally Sponsored Research or Development

This invention was made with U.S. Government support under grant number R01 HL069929, R01-AI080455, R01-AI100288, R01-AI101406, P01-CA023766 and P01-CA023766 from the National Institutes of Health and Contract HHSN272200900059C from the U.S. National Institute of Allergy and Infectious Disease. The government has certain rights in the invention.

### Technical Field of the Invention

The present invention relates generally to graft versus host disease (GVHD). More particularly, the present invention relates to a therapeutic composition for use in treating a graft versus host disease (GVHD) in a subject following bone marrow transplant (BMT) or hematopoietic stem cell transplant (HSCT).

### Background of the Invention

Despite continuing improvements in outcomes of patients undergoing allogeneic bone marrow transplant (allo BMT), GVHD continues to be a leading cause of mortality in this population'. Modern immune suppression strategies are only partially effective at preventing GVHD and simultaneously increase the risks for infections and disease recurrence. Strategies that reduce GVHD but leave immune function intact can thus potentially improve outcomes. One such strategy is to target the complex community of microbes that reside within our intestinal tracts, collectively termed the intestinal microbiota.

A relationship between the microbiota and GVHD has long been suspected but is still not well understood. Mice transplanted in germ-free conditions² or receiving gut-decontaminating antibiotics³ develop less severe GVHD. Clinical studies initially suggested a benefit from near-total bacterial decontamination^{4,5}, but later showed no clear benefit⁶⁻⁸ and this approach was discontinued in the early 1990s⁹. Partial gut decontamination continues to be practiced but little is known regarding optimal antibiotics. One study found the addition of metronidazole to ciprofloxacin led to a significant reduction in acute GVHD, suggesting that anaerobic bacteria may contribute to GVHD pathogenesis¹⁰.

More recent studies, however, indicate that this approach may not be ideal. The administration of metronidazole during allo BMT was associated with expansion of vancomycin-resistant Enterococcus within the intestinal tract, which in some patients preceded enterococcal bacteremia¹¹. Other studies have found that obligate anaerobes in the intestine, in particular Clostridial species, are important mediators of intestinal homeostasis and prevent inflammation by upregulating intestinal regulatory T cells¹².

Recently it was reported that increased bacterial diversity at the time of engraftment was associated with improved overall survival following allo BMT and reduced transplant-related mortality¹³. The population studied, however, was heterogeneous and in particular included 45% patients who received a T-cell depleted allograft. Recipients of this type of transplant are at much lower risk of developing GVHD. Likely because of inadequate numbers of patients and heterogeneity, it was not possible to determine the subcategories of non-relapse mortality associated with low diversity, which include GVHD, infection and organ failure. US2014/341921 A1 discloses the treatment! prevention of rejection in organ transplantation, such as GVHD following bone marrow transplantation, using human commensal bacteria. Jenq et al., 2012, J. Exp. Med, 209:903-911 discloses that intestinal inflammation secondary to GVHD is associated with shifts in the composition of intestinal microbiota.

Thus the need exists for a treatment that exploits the relationship between intestinal microbiota and GVHD.

### Summary of the Invention

In an aspect, the invention provides therapeutic composition for use in treating a graft versus host disease (GVHD) in a subject following bone marrow transplant (BMT) or hematopoietic stem cell transplant (HSCT), wherein the therapeutic composition comprises one or more purified populations of bacteria selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* and combinations or mixtures thereof. In another aspect, the invention provides a therapeutic composition for use in preventing, reducing the likelihood of, and/or treating a graft versus host disease (GVHD) in a subject undergoing bone marrow or hematopoietic stem cell transplant, wherein the therapeutic composition comprises one or more purified populations of bacteria selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* and combinations or mixtures thereof. In another aspect, the invention provides a method for screening a subject for risk of developing GVHD following bone marrow transplant (BMT) or hematopoietic stem cell transplant (HSCT), the method comprising determining the abundance of a bacterial species of the order *Clostridiales* in a sample of fecal material from the subject, wherein the subject is at an increased risk for GVHD if the abundance of said *Clostridiales* in said sample of fecal material is less than the abundance of said *Clostridiales* in a sample of fecal material from a subject who is not at risk for developing GVHD, and wherein said *Clostridiales* species is selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli* and combinations or mixtures thereof. The present disclosure is based on the observation that graft versus host disease correlates with major changes in intestinal microbiota that occur during bone marrow and/or hematopoietic stem cell transplant suggesting that commensal bacteria can be predictors and modulators of GVHD risk and severity.

The present disclosure therefore relates to methods and compositions for preventing the loss of or restoring mammalian bacterial gastrointestinal microbiota in a subject during bone marrow or hematopoietic stem cell transplant in order to prevent, reduce the severity or treat GVHD. The disclosure encompasses several approaches or a combination thereof for preventing loss of relevant bacteria in the first instance, for restoring bacteria in a subject that has sustained loss of protective bacteria and supporting the endogenous populations or the repopulated bacteria. The approaches include:
(1) selection of antibiotics that have lower activity against obligate anaerobic bacteria as a way to preserve and prevent loss of endogenous protective bacteria;
(2) providing prebiotics that support the growth of the endogenous population or repopulated beneficial bacteria; and
(3) where beneficial bacteria have already been lost, providing a probiotic, that is, administering to the subject a therapeutically effective amount of a therapeutic composition comprising one or more beneficial bacteria to repopulate the gastrointestinal tract.

The disclosure relates to a method for restoring gastrointestinal bacteria that has been lost, for example, as the result of exposure to antibiotics with high activity against anaerobes, comprising administering to a subject in need of such treatment, an effective amount of at least one bacteria from the order *Clostridiales,* or combinations thereof. The bacteria may be administered orally. Alternatively, bacteria can be administered rectally, for example, by enema.

The present disclosure relates to compositions for the reduction of graft versus host disease (GVHD) and GVHD-related mortality. It is based on the observation that there is a change in the microbiota of the gut that correlates with GVHD-related mortality. In particular, the presence of certain bacterial species including some organisms that ferment xylose, raffinose, cellobiose or melizitose is particularly effective in reducing GVHD-related mortality.

The disclosure relates to a method of reducing the risk of developing graft versus host disease (GVHD) and/or treating GVHD in a subject undergoing bone marrow transplant or hematopoietic stem cell transplant, the method comprising administering to the subject a therapeutically effective amount of a therapeutic composition comprising one or more bacteria from the order *Clostridiales,* wherein the composition
(i) stimulates the growth or activity of one or more bacterial taxa which are under-represented in microbiota of the subject either before transplant or following transplant; or
(ii) inhibits the growth or activity of one or more bacterial taxa which are over-represented in microbiota of the subject.

Disclosed herein is a method for reducing the likelihood, incidence or severity of GVHD in a subject, the method comprising administering to the subject a composition comprising at least one species of the order *Clostridiales.* The organism may comprise a 16SrDNA with the nucleotide sequence of GenBank X94966, a nucleotide sequence selected from SEQ ID NOS: 1, 3, 4, 5, 7, 8, 9, 12 and 15 or a sequence with about 98% to 100% identity to any of said sequences; for example about 99-100%; in for example about 99.5-100%. The therapeutic composition may comprise bacteria selected from genuses *Blautia, Ruminococcus, Eubacterium, Holdemania,* and *Clostridium.* The bacteria may be selected from the group consisting of *Ruminococcus obeum, Clostridium hathewayi, Eubacterium desmolans, Dorea longicatena, Ruminococcus lactaris (Blautia producta), Eubacterium* contortum *Ruminococcus faecis, Holdemania filiformis, Clostridium sordelli* and combinations or mixtures thereof.

The *Blautia* species may be *Blautia producta.*

Disclosed herein is a therapeutic composition comprising a *Clostridiales* species. The organism may comprise a 16SrDNA with the nucleotide sequence of GenBank X94966, a nucleotide sequence selected from SEQ ID NOS: 1, 3, 4, 5, 7, 8, 9, 12 and 15 or a sequence with about 98% to 100% identity to any of said sequences; for ex-ampe about 99-100%; for example about 99.5-100%. The therapeutic composition may comprise bacteria selected from genuses, *Blautia, Ruminococcus, Eubacterium, Holdemania,* and *Clostridium.* The bacteria may be selected from the group consisting of *Ruminococcus obeum, Clostridium hathewayi, Eubacterium desmolans, Dorea longicatena, Ruminococcus lactaris (Blautia producta), Eubacterium* contortum *Ruminococcus faecis, Holdemania filiformis, Clostridium sordelli* and combinations or mixtures thereof.

Disclosed herein is a method for reducing the likelihood of or preventing GVHD, wherein a composition comprising at least one species of *Clostridiales* is administered to the subject from about 1 week to about 2 weeks before allo BMT, from about 1 day to about 2 weeks before all BMT, and from about 7-10 days before allo BMT.

A method for reducing the risk, incidence or severity of graft versus host disease (GVHD) in a subject undergoing a bone marrow transplant (BMT) or hematopoietic stem cell transplant (HSCT), the method comprising administering to the subject a therapeutically effective amount of oral vancomycin or ampicillin when the subject has been treated for neutropenic fever with an intravenous antibiotic selected from the group consisting of metronidazole, piperacillin-tazobactam (pip-tazo), imipenem.

A method for reducing the risk of developing graft versus host disease (GVHD) in a subject following a bone marrow transplant (BMT) or hematopoietic stem cell transplant (HSCT), the method comprising: determining the abundance of *Akkermansia muciniphila* in a sample of fecal material from the subject; and administering a therapeutically effective amount of an antibiotic selected from ampicillin and oral vancomycin to the subject when the abundance of *Akkermansia muciniphila* is above from 1% to 10%, wherein administration of the antibiotic reduces abundance of *Akkermansia muciniphila* and the risk of GVHD is reduced or eliminated. Abundance of *Akkermansia muciniphila* in said sample above 2% may indicate risk of developing GVHD. The abundance of *Akkermansia muciniphila* is determined prior to transplant, following antibiotic treatment for transplant-related neutropenic fever or both.

### Brief Description of the Drawings

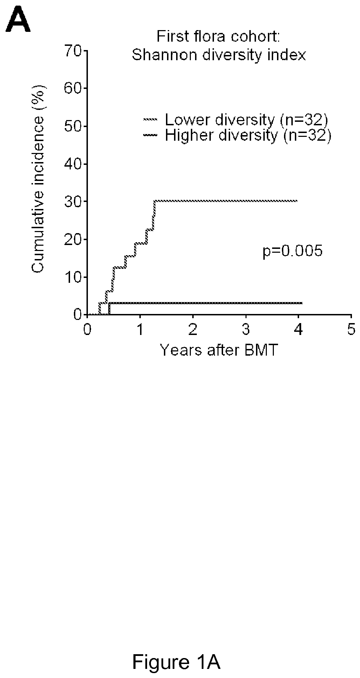

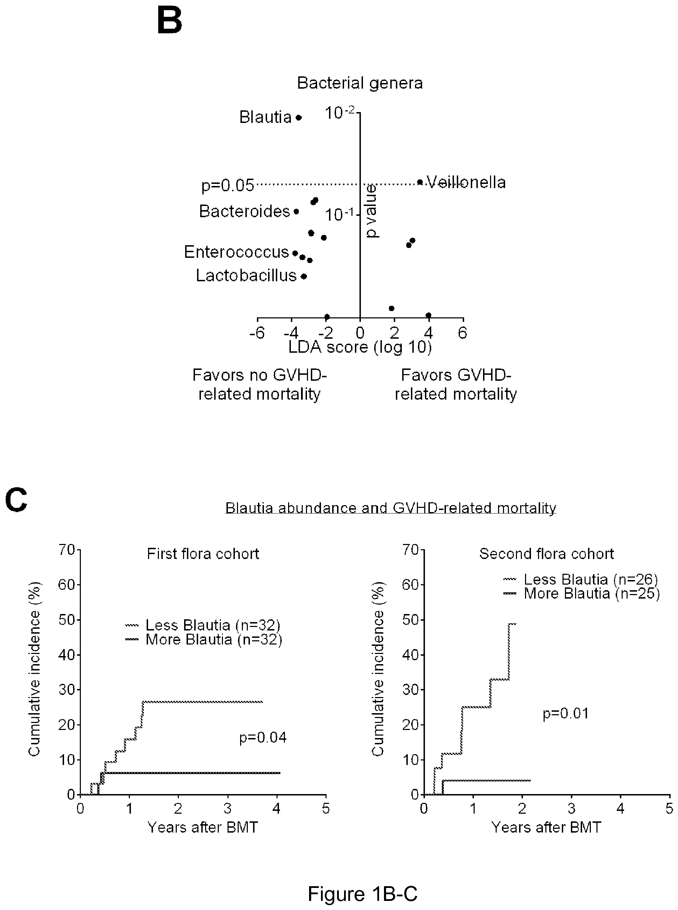

**Figure 1** shows that changes in the intestinal flora are associated with differences in GVHD-related mortality. **A)** Bacterial composition of stool samples from 64 patients in an initial flora cohort was analyzed by 16S gene sequencing and bacterial diversity was quantified using the Shannon index. Patients were stratified by the median Shannon index value (2.6) and analyzed for cumulative incidence of GVHD-related mortality. Bottom half indicates patients with Shannon index below the median, while top half indicates patients with Shannon index above the median. **B)** Associations of bacterial genera with GVHD-related mortality outcomes were quantified by LEfSe analysis. **C)** Patients from the first flora cohort and a subsequent flora cohort were stratified by median *Blautia* abundance (0.05% in both) and analyzed for incidence of GVHD-related mortality.

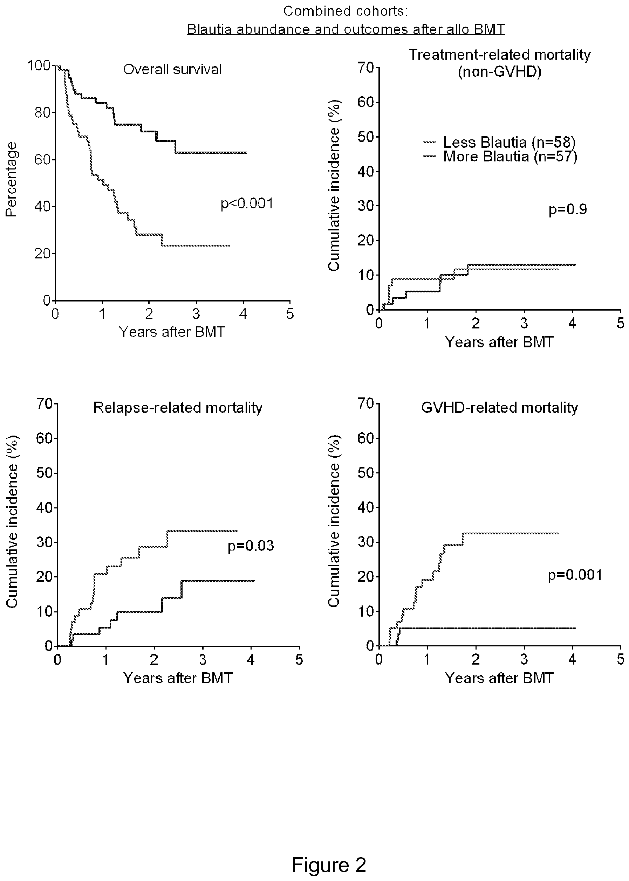

**Figure 2** shows association of *Blautia* abundance and outcomes after allo BMT. Patients from the two flora cohorts were combined and stratified by *Blautia* abundance below or above 0.05%, and evaluated for the indicated outcomes; similar results were seen for each individual cohort.

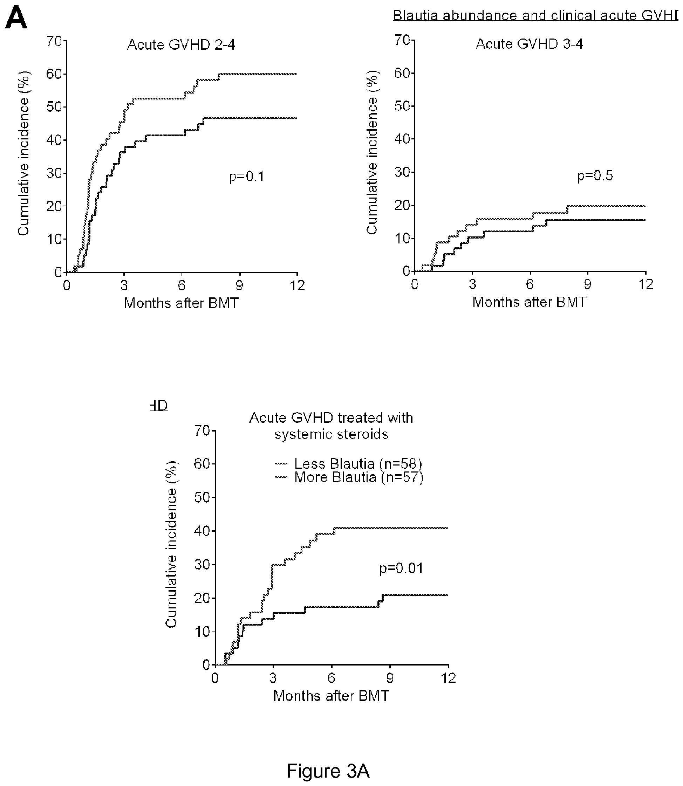

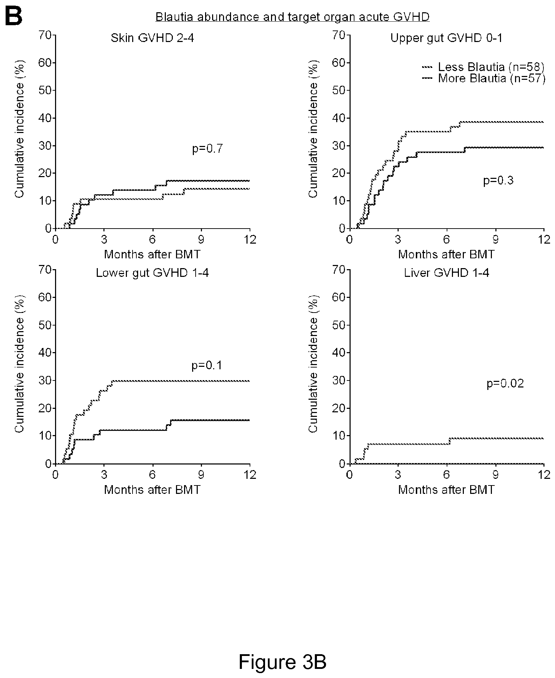

**Figure 3** shows the association of *Blautia* abundance with clinical acute GVHD. **A)** Patients were stratified by *Blautia* abundance below or above 0.05%, and evaluated for development of the indicated severity grades of acute GVHD, as well as acute GVHD that required systemic therapy with corticosteroids. **B)** Patients were evaluated for development of acute GVHD in typical target organs.

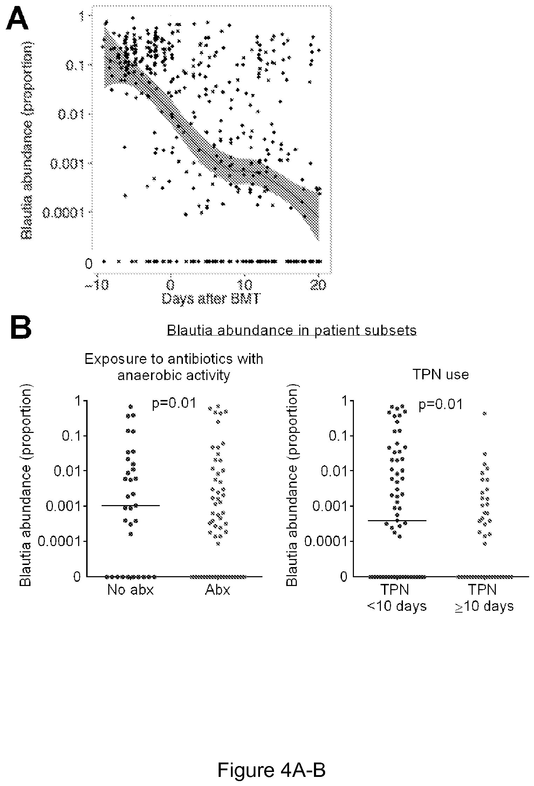

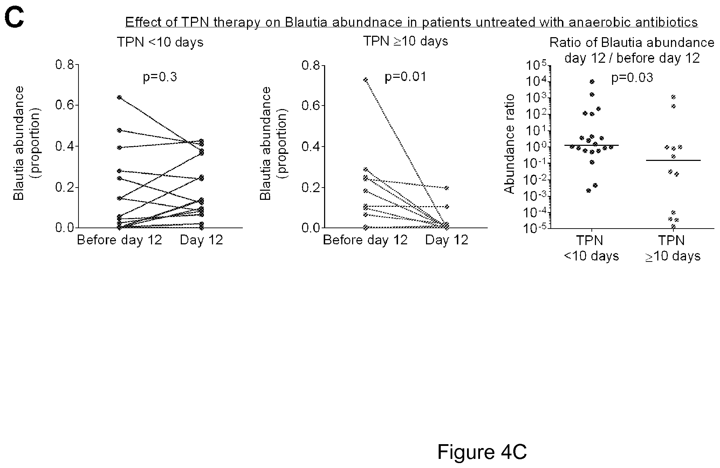

**Figure 4** Identifying potential determinants of *Blautia* abundance in allo BMT patients. **A)** *Blautia* abundances in all available stool samples from both flora cohorts were evaluated and plotted. An abundance trend was constructed using moving average filtering (solid blue line; 95% confidence intervals shown in gray). **B)** Patients were subsetted by exposure to antibiotics with anaerobic coverage prior to sample collection, and duration of TPN therapy, and *Blautia* abundance was evaluated. **C)** A subset of patients who were not exposed to antibiotics with anaerobic coverage prior to sample collection were further subsetted by length of TPN therapy and evaluated for *Blautia* abundance in a stool sample prior to day 12 compared to on day 12.

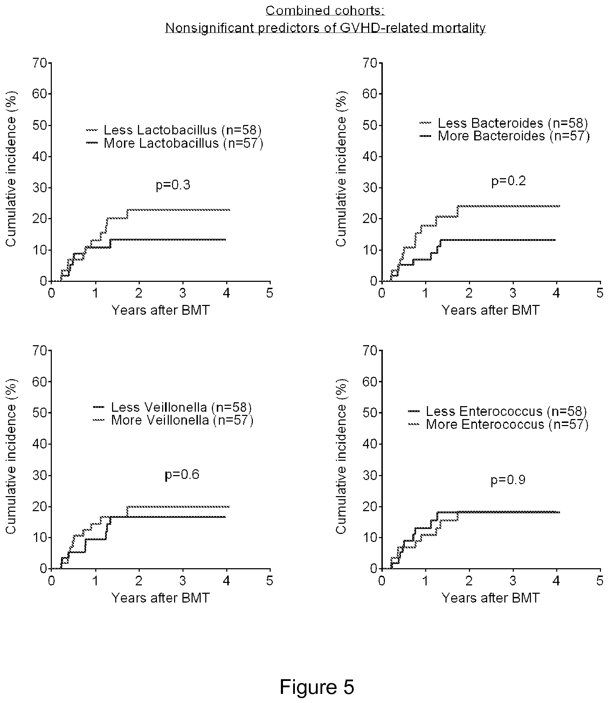

**Figure 5** Evaluation of other factors potentially associated with GVHD-related mortality are not strongly predictive in this patient population. Patients from combined cohorts were stratified by the median abundance of the indicated bacterial genera (Lac*tobacillus* 0.2%, *Bacteroides* 0.02%, *Veillnoella* 0.03%, *Enterococcus* 1.3%) and evaluated for incidence of GVHD-related mortality.

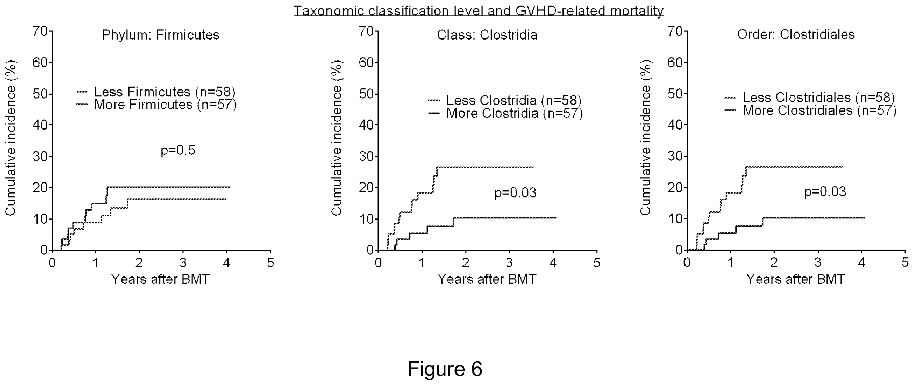

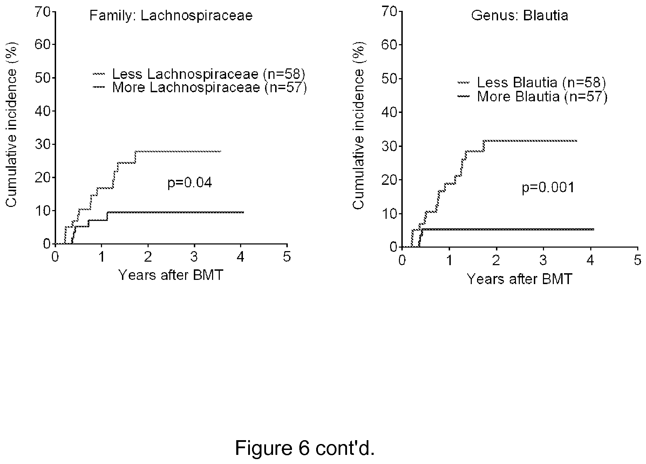

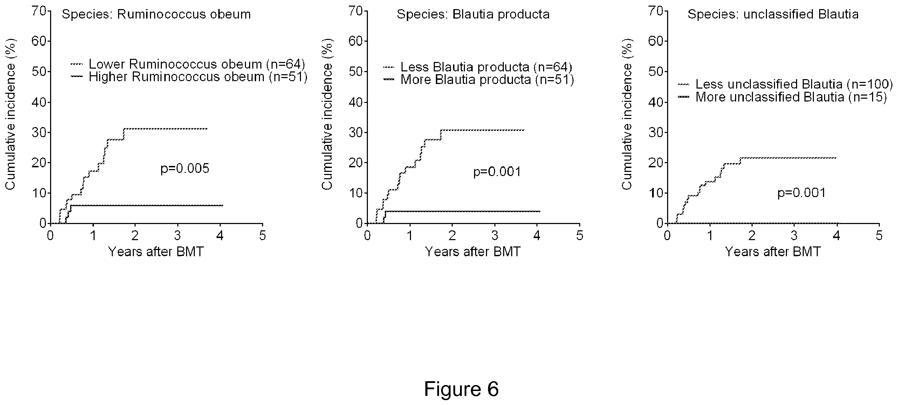

**Figure 6** Evaluation of *Blautia* and related bacteria by taxonomic classification levels and association with GVHD-related mortality. Patients from combined flora cohorts were stratified by the median abundance of the indicated bacterial taxons (*Firmicutes* 95%, *Clostridia* 3.5%, *Clostridiales* 2.9%, *Lachnospiraceae* 0.2%, *Blautia* 0.05%) and evaluated for incidence of GVHD-related mortality. At the species level, the median abundance was 0% for all three taxons, resulting in unequal numbers of patients following stratification. *Ruminococcus obeum* is considered to be a member of the genus *Blautia* by 16srRNA gene similarity but has not been officially renamed according to the Bacteriological Code because it has not been possible to deposit this species in a second culture collection in a second country.

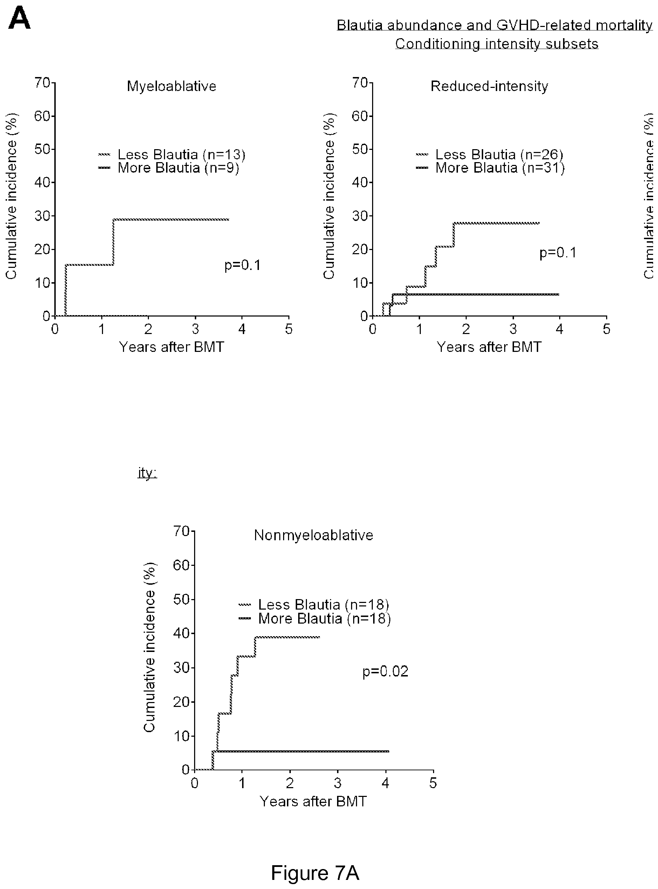

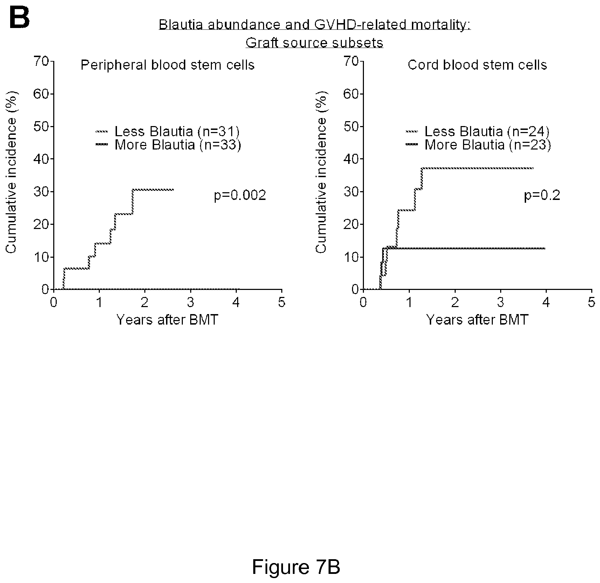

**Figure 7** Evaluating the association of *Blautia* abundance and GVHD-related mortality in conditioning intensity and graft source subsets. **A)** Patients were subsetted by conditioning intensity, stratified by *Blautia* abundance below or above 0.05%, and evaluated for incidence of GVHD-related mortality. **B)** Patients were subsetted by graft source, stratified by *Blautia* abundance below or above 0.05%, and evaluated for incidence of GVHD-related mortality.

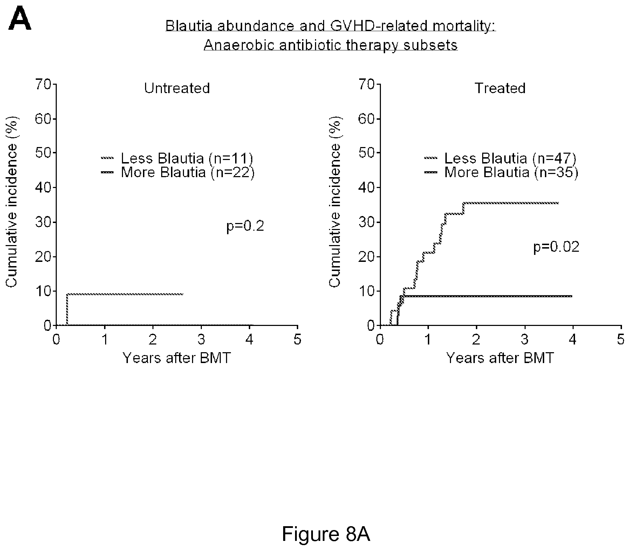

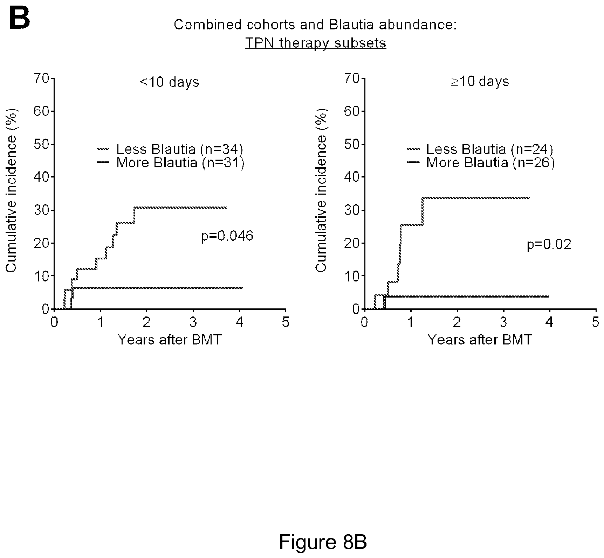

**Figure 8** Evaluating the association of *Blautia* abundance and GVHD-related mortality in antibiotic and TPN therapy subsets. **A)** Patients were grouped by exposure to antibiotics with anaerobic activity, stratified by *Blautia* abundance below or above 0.05%, and evaluated for incidence of GVHD-related mortality. **B)** Patients were subsetted by duration of TPN therapy, stratified by *Blautia* abundance below or above 0.05%, and evaluated for incidence of GVHD-related mortality.

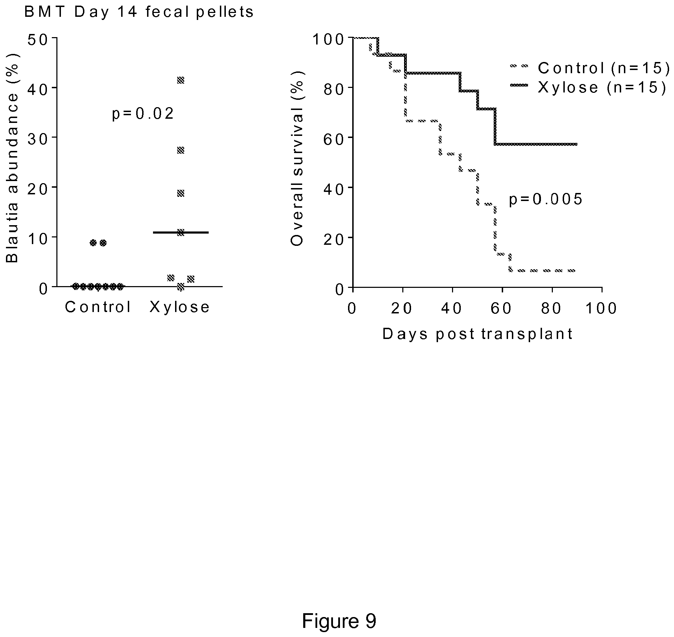

**Figure 9** shows that targeted nutritional support of *Blautia* results in increased abundance in the setting of GVHD and reduced GVHD severity. The drinking water of C57BL/6 mice was treated with xylose (10 g/L), a sugar that is commonly fermented by *Blautia,* beginning one week before BMT. Left: Intestinal abundance of *Blautia* was evaluated on day 14 after BMT, results of a single experiment. Right: Mice were followed for development of clinical GVHD (combined results of 2 experiments).

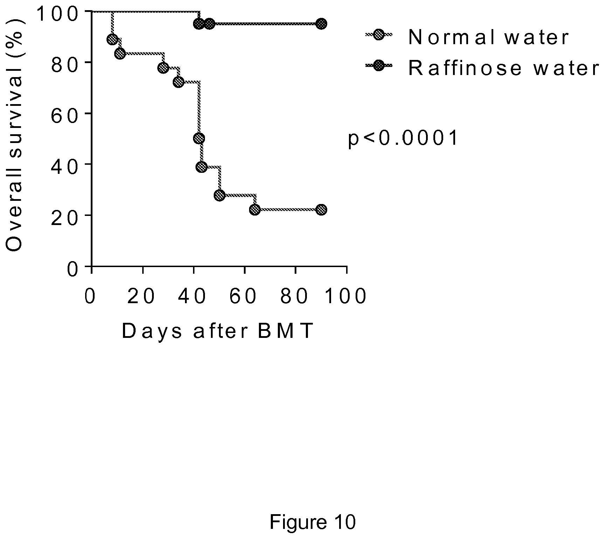

**Figure 10** shows that targeted nutritional support with raffinose reduces GVHD severity. The drinking water of C57BL/6 mice was treated with raffinose (10 g/L), a sugar that is commonly fermented by *Blautia,* beginning one week before BMT. Mice were followed for development of clinical GVHD (combined results of 3 experiments).

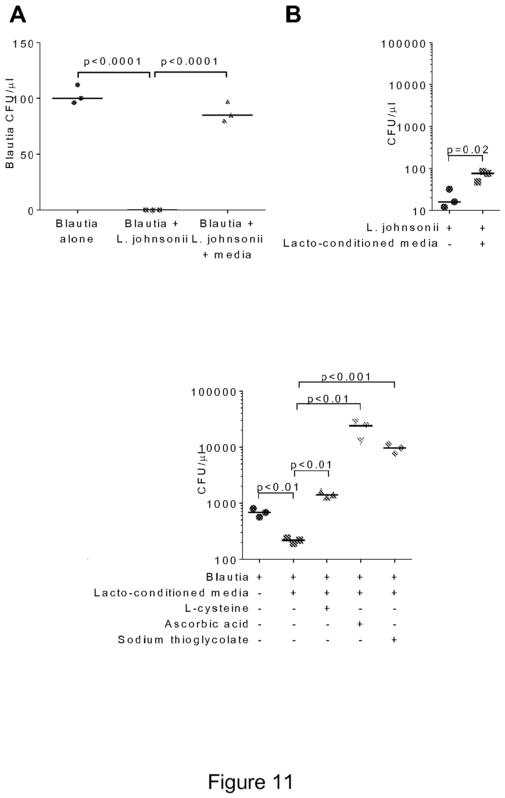

**Figure 11** shows that growth of *Blautia in vitro* is suppressed by factors released by *Lactobacillus johnsonii* under starvation conditions. **A)** Murine *Blautia* was grown in peptone yeast glucose broth either alone, or with murine *L. johnsonii* in conditions of limited media or ample media, and viable colonies were quantified. B) *L. johnsonii* was allowed to grow in media to plateau phase, then sterile filtered to generate Lacto-conditioned media. Effects of Lacto-conditioned media on the viability of *L. johnsonii* and murine *Blautia* were evaluated when mixed with fresh media in a 1:1 ratio. Effects of the addition of 3 reducing agents indicated was also evaluated.

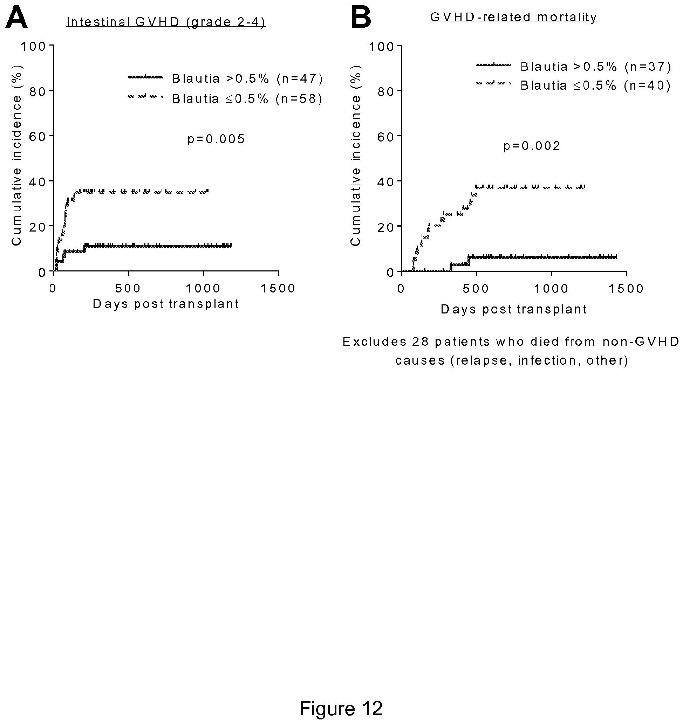

**Figure 12** shows that intestinal *Blautia* abundance predicts for GVHD in humans. **A)** A cohort of 105 patients, stratified by abundance of *Blautia,* was evaluated for incidence of intestinal GVHD. **B)** The same cohort stratified by *Blautia* abundance was evaluated for GVHD-related mortality.

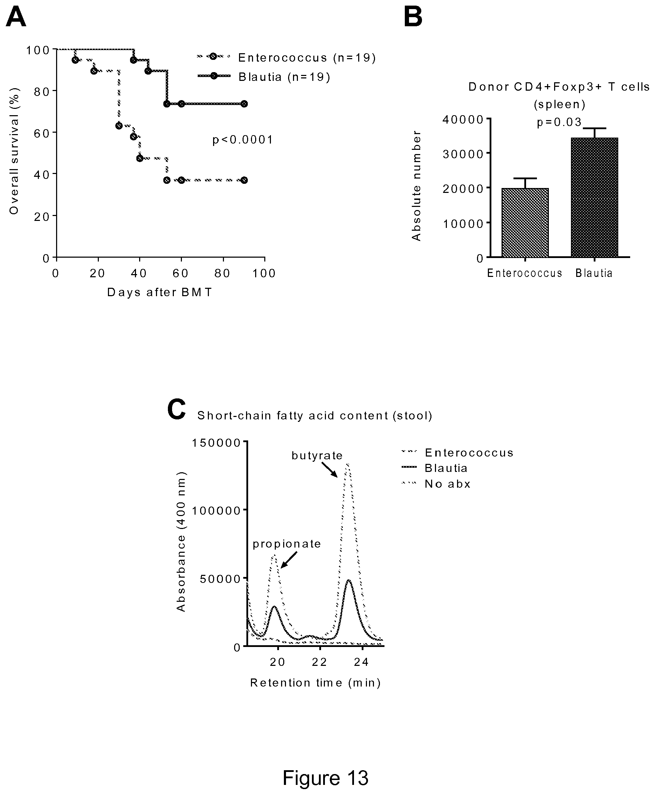

**Figure 13** shows that administration of *Blautia* to mice mitigates GVHD severity. Mice were treated orally with a 2 day course of vancomycin, a 2 day course of ampicillin, then inoculated 5 and 7 days later with *Blautia* or *Enterococcus* of murine origin, and then 2 weeks later irradiated and transplanted with B10.BR bone marrow and T cells. **A)** Overall survival, combined results from 2 experiments. **B)** Flow cytometric evaluation of T cell populations, results of a single experiment, mice harvested on BMT day 14 n=4/group. **C)** Mice were treated with antibiotics and bacteria as in **A** but without BMT, and stool was evaluated for short chain fatty acid content by HPLC two weeks after bacterial introduction.

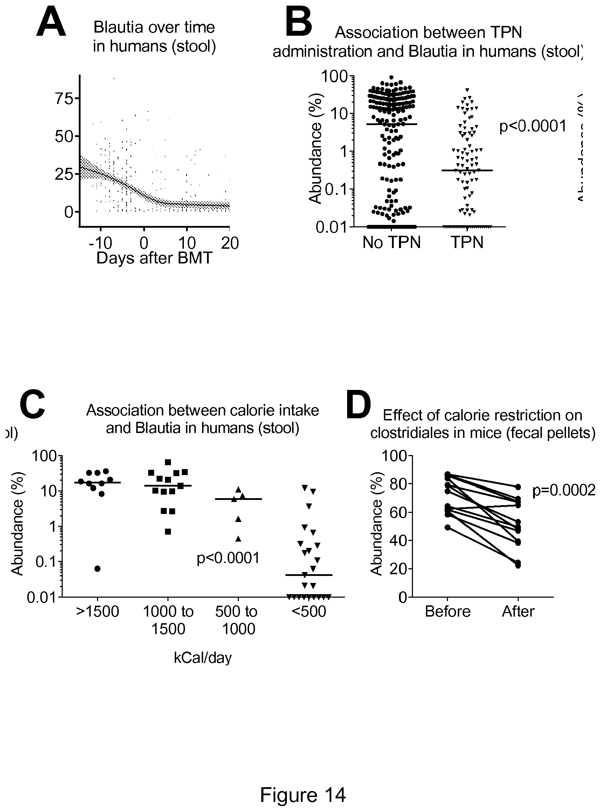

**Figure 14** shows that reduced nutritional intake appears to drive loss of *Blautia* in allo BMT recipients. **A)** Ninety-four allo BMT patients were evaluated weekly for changes in the intestinal microbiota during transplant hospitalization. *Blautia* abundance is depicted by a solid black line with 95% confidence bands shown in gray constructed using moving average filtering. **B)** *Blautia* abundance was assayed in stool samples collected from allo BMT patients either not receiving or receiving total parenteral nutrition (TPN), a surrogate marker for poor oral intake. **C)** Daily nutritional and microbiota analysis was collected in 5 allo BMT patients during transplant hospitalization, and abundance of *Blautia* analyzed for stool samples stratified by kCal consumption. **D)** Stool abundance of *Clostridiales* (the order to which *Blautia* belongs) was assayed in mice before and after one week of 25% reduction in food consumption.

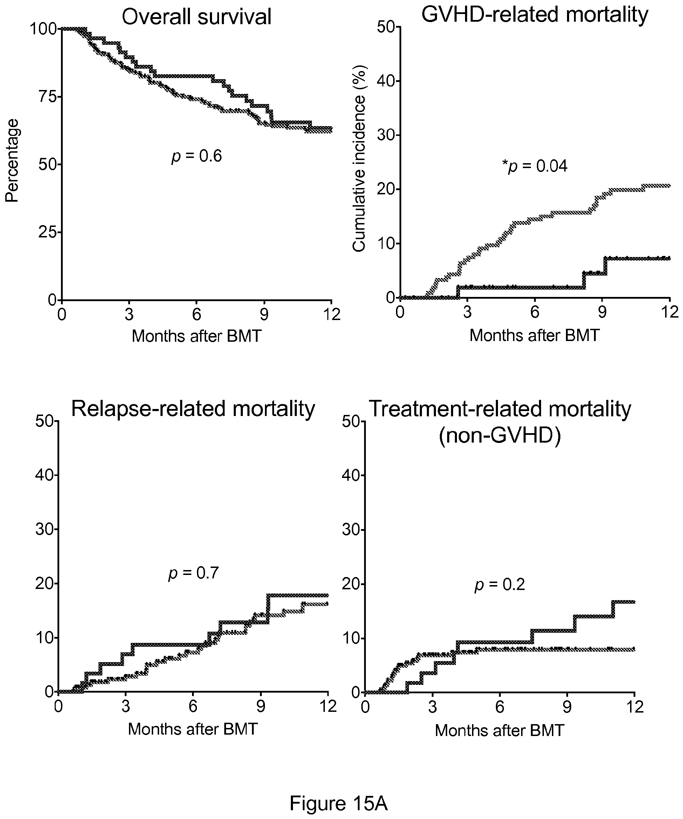

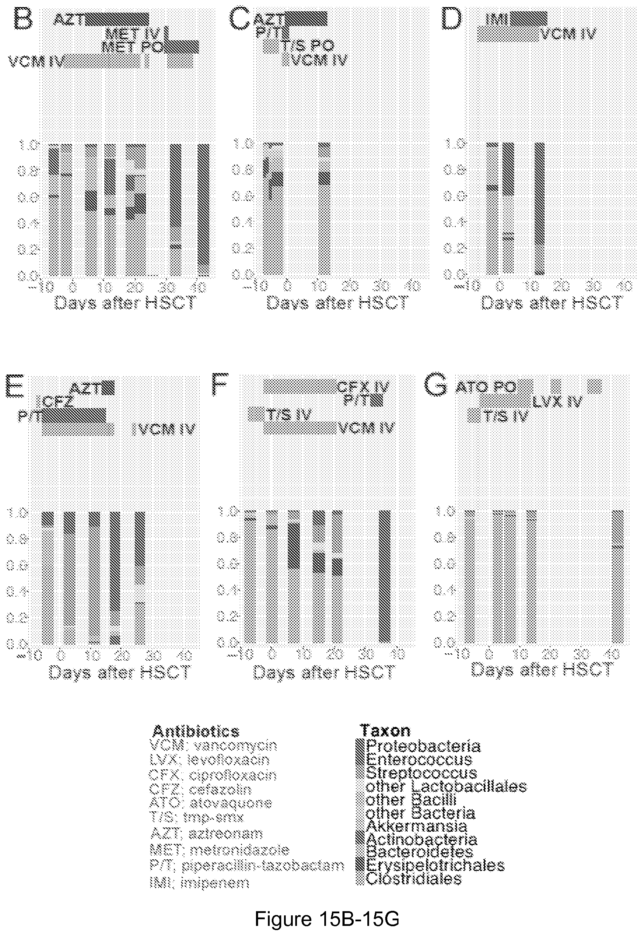

**Figure 15** (1 from Shono paper) shows that clinical use of anaerobe-active antibiotics for neutropenic fever is associated with increased GVHD-related mortality. **(A)** A retrospective cohort was identified of 283 adult patients who received non-T cell depleted allo-HSCT at our center from 1994 to 2013 and received antibiotics for neutropenic fever. Patients were stratified by exposure to antibiotics with significant activity against anaerobes. Outcomes indicated were depicted by Kaplan-Meier plots and curves compared by the logrank test. **(B** to **G)** Six representative clinical cases with time courses depicted of fecal microbiota composition and administration of antibiotic treatments during the course of allo-HSCT. The dynamics of flora composition that occur in the setting of treatment are shown for aztreonam **(B** and **C),** imipenem **(D),** pip/tazo **(E** and **F),** metronidazole **(B),** and minimal flora perturbing antibiotics **(G). (H)** Change in abundance of Clostridiales in paired fecal samples from patients undergoing allo-HSCT collected prior to and following treatment with the indicated antibiotics.

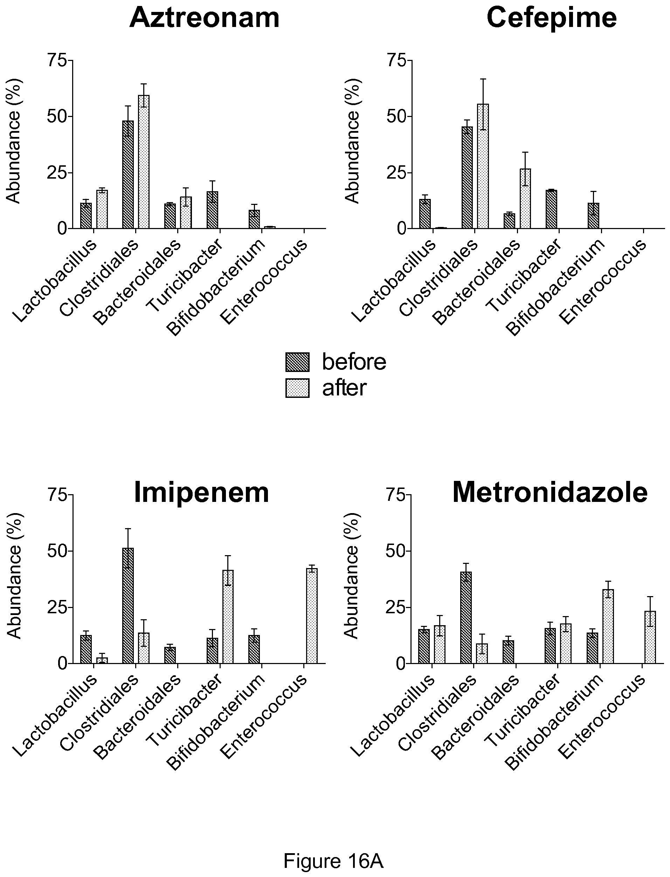

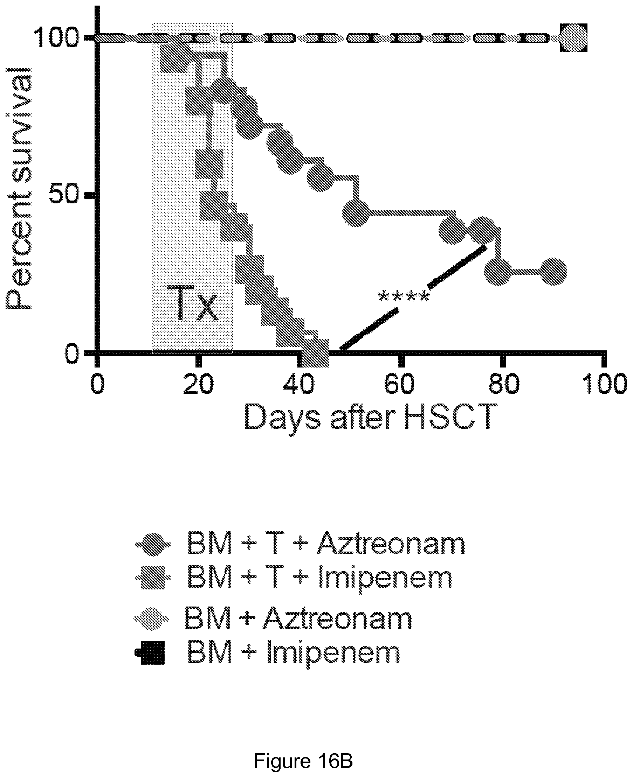

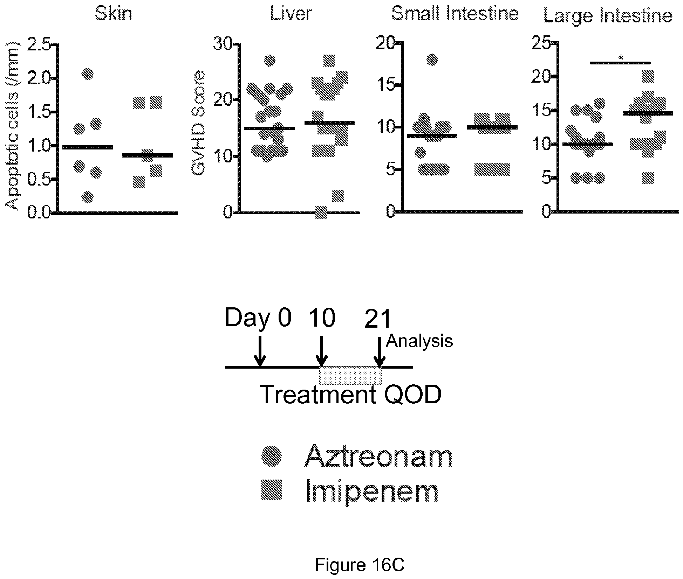

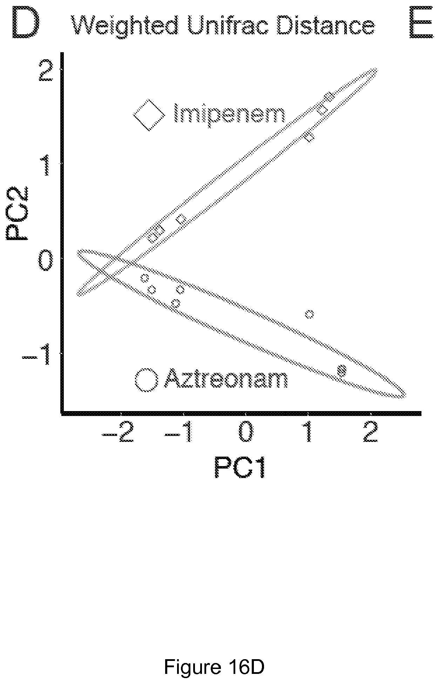

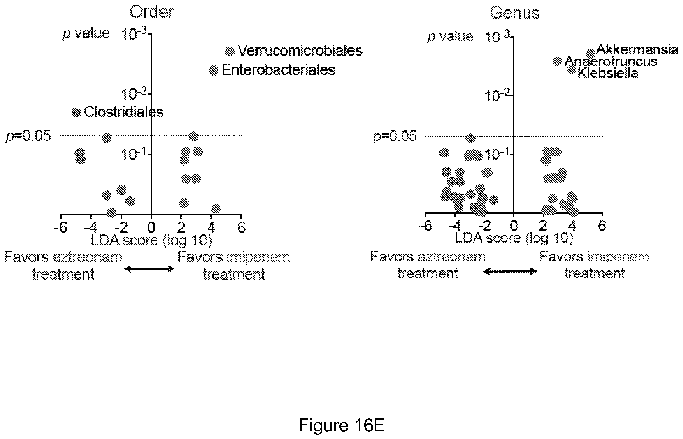

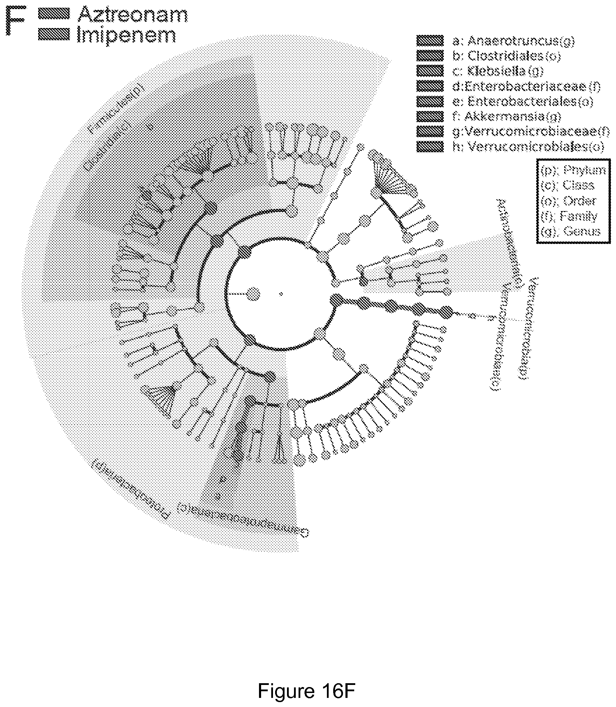

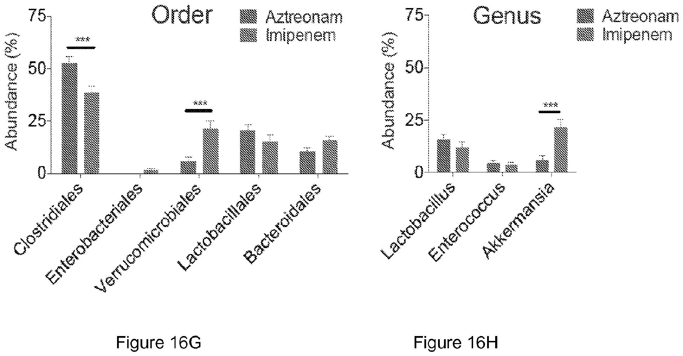

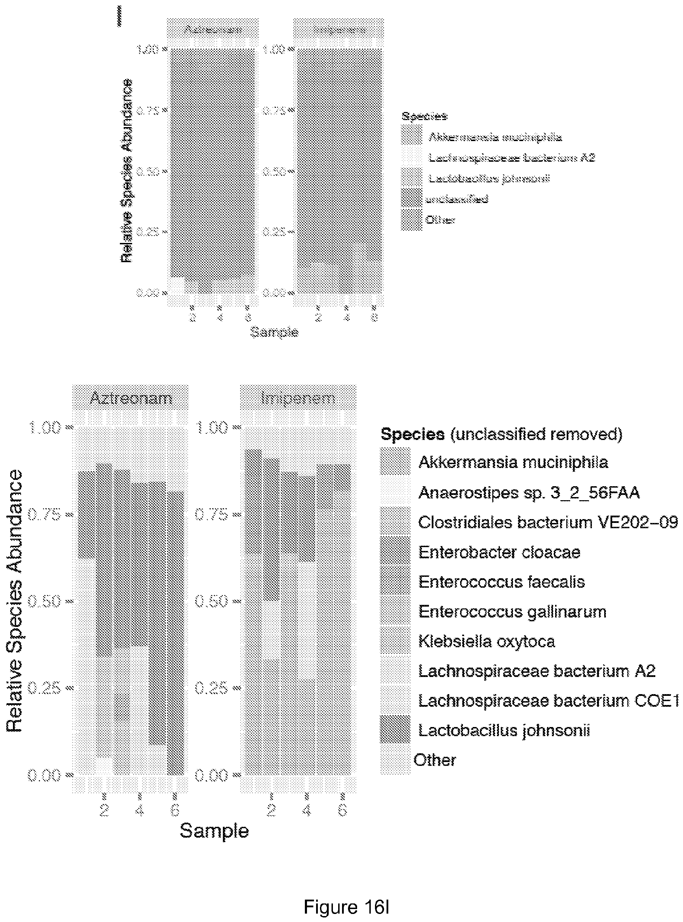

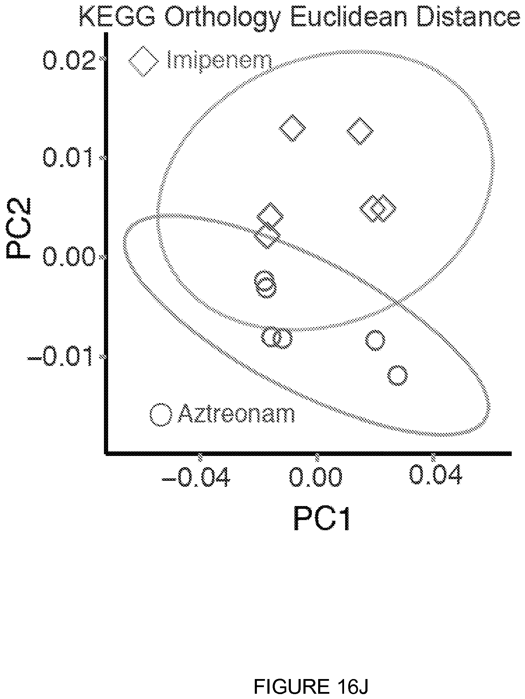

**Figure 16** Imipenem treatment, compared to aztreonam treatment, supresses anaerobic commensals and elevates GVHD severity in mice. **(A)** Flora composition analysis using 16S rRNA sequencing prior to and after treatment with the indicated antibiotics in healthy C57BL/6 mice. Mice were treated with subcutaneous (SC) injections of each antibiotic twice a day for two days (500 mg/kg for pip/tazo and 100 mg/kg for others) and stool samples were collected the following day. Values represent mean ± SEM (n = 3-4). *, P < 0.05; **, P <0.01; ****, P < 0.0001. Data are representative of two independent experiments. **(B** to **J)** Lethally irradiated 129S1 recipients were transplanted with MHC matched C57BL/6 T-cell depleted bone marrow (TCD-BM) cells and 1 × 10⁶ C57BL/6 T cells. Control mice received TCD-BM only. Recipients were treated with imipenem or aztreonam (100 mg/kg, SC, 3 times a week from day 10 to day 24 following allo-HSCT). **(B)** Comparison of overall survival, with combined data from three independent experiments (n = 15-43). ****, P < 0.0001. **(C)** Mice with GVHD treated with antibiotics were sacrificed on day 21 and GVHD histology scores in target organs were quantified by a blinded pathologist. Data are combined from three independent experiments (n = 5-20). *, P < 0.05. **(D)** Stool samples obtained from mice treated with imipenem or aztreonam similarly to those in B were collected on day 21 and analyzed by 16S rRNA gene sequencing, followed by principal coordinate analysis of weighted and normalized UniFrac distances. **(E** and **F)** Differential taxonomic abundance between aztreonam treated and imipenem treated recipients was analyzed by **(E)** linear discriminate analysis coupled with effect size measurements (LEfSe) and **(F)** by LEfSe projected as a cladogram. Data are representative from more than five independent experiments in D to F. **(G** and **H)** Comparisons of bacterial abundance at the phylogenetic levels of **(G)** order, and **(H)** genus are shown. Data are combined from 6 independent experiments (n = 32-36). ***, P < 0.001. **(I)** Stool samples collected from mice with GVHD treated with antibiotics were collected on day 21, and evaluated by metagenomic shotgun sequence analysis, comparison of bacterial species abundance determined by taxonomy. Numbers 1 through 6 along the x-axis represent the individual subjects. **(J)** Principal component analysis of quantification of sequence reads from KEGG gene orthologs comparing samples from mice treated with aztreonam and imipenem.

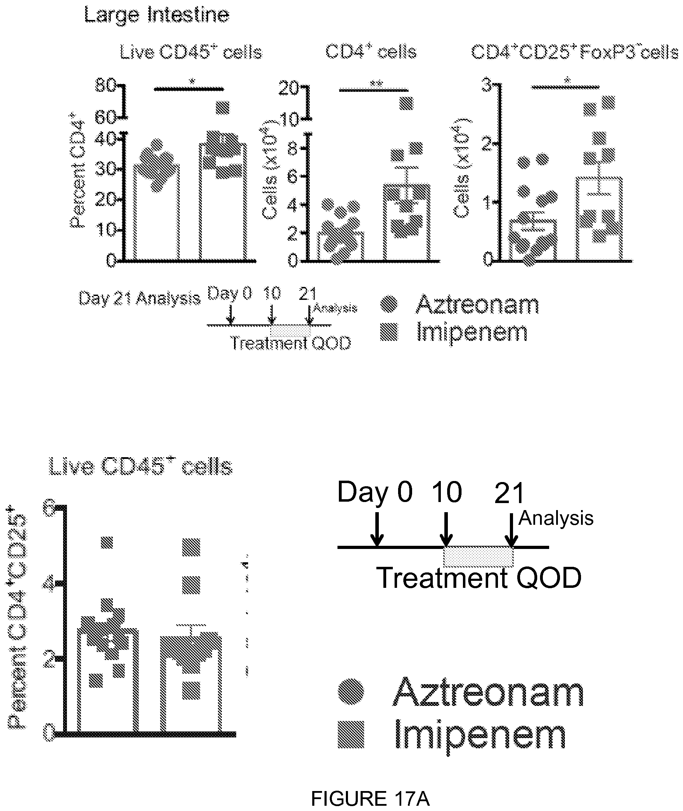

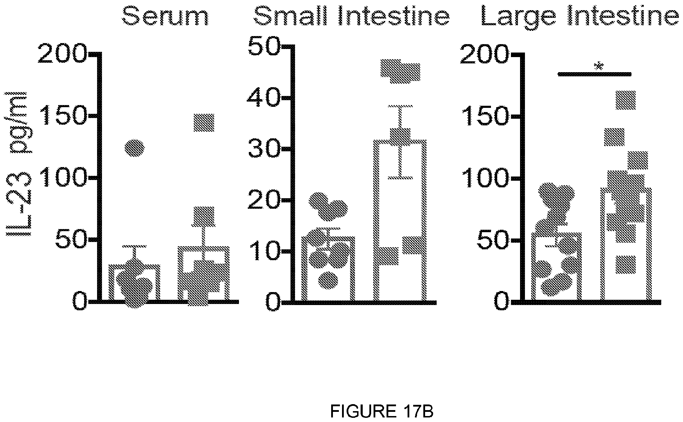

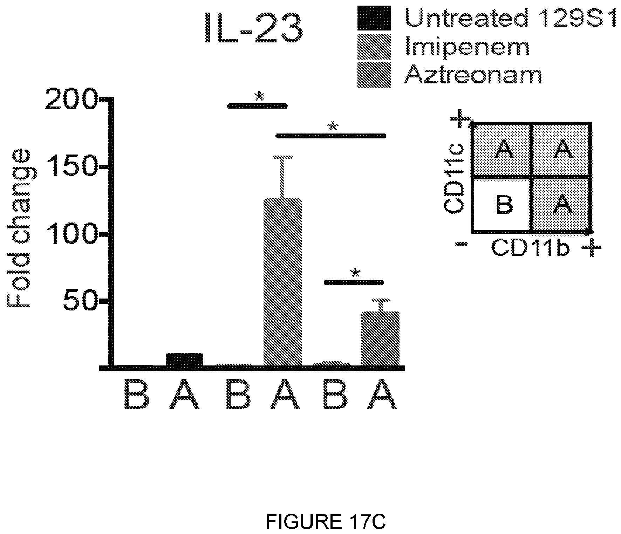

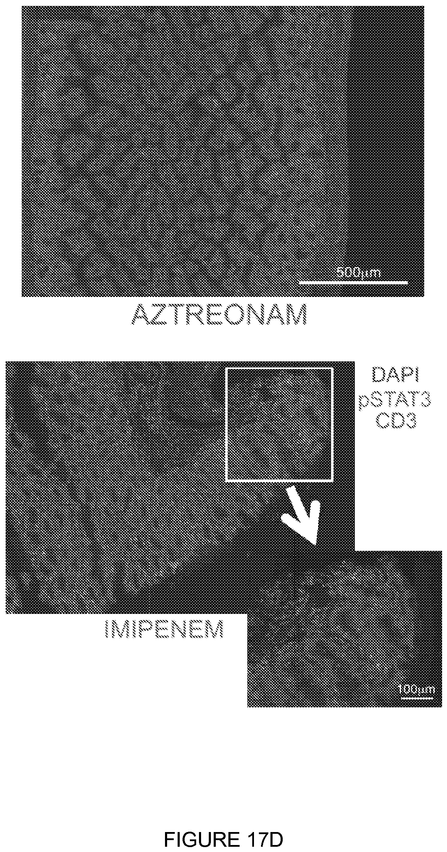

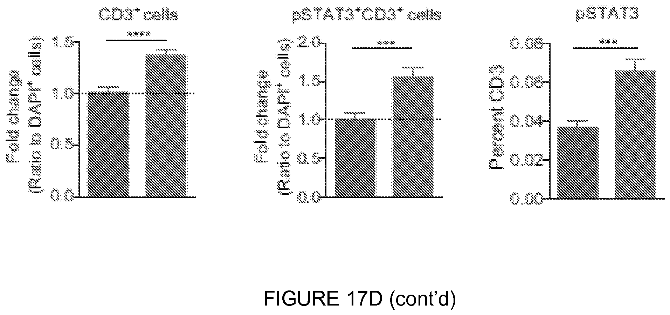

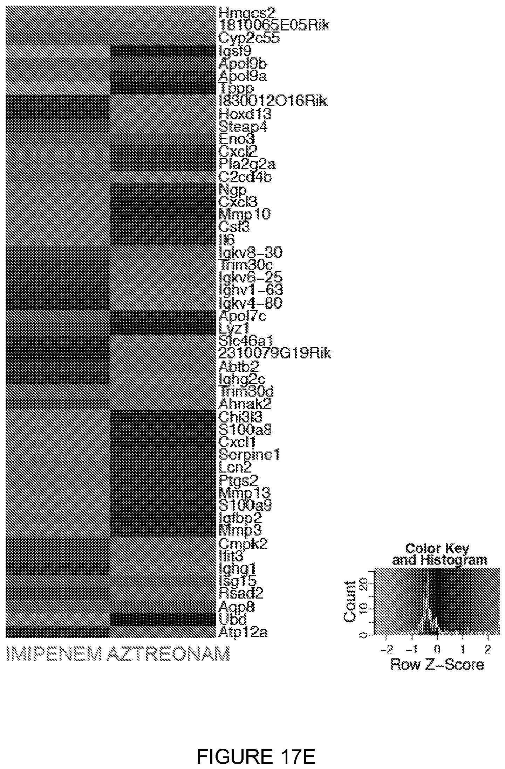

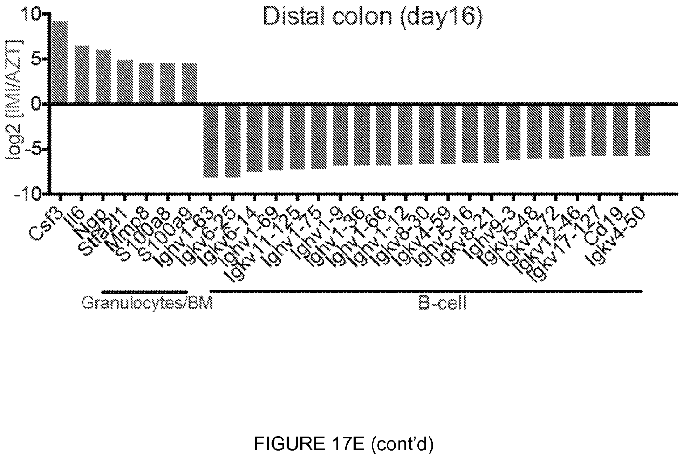

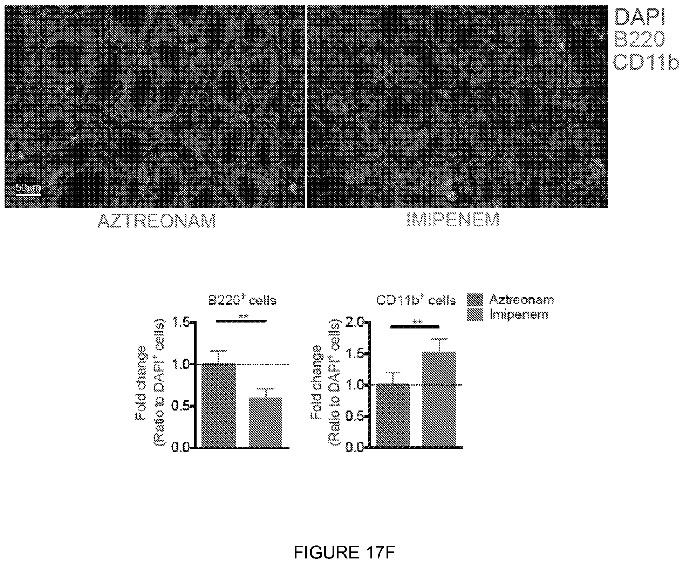

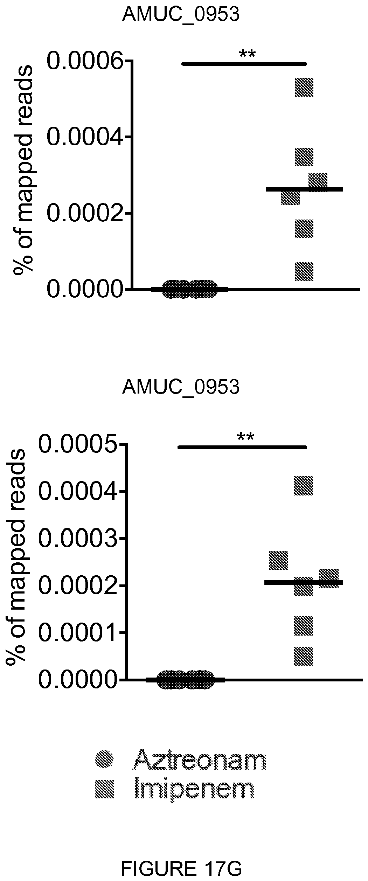

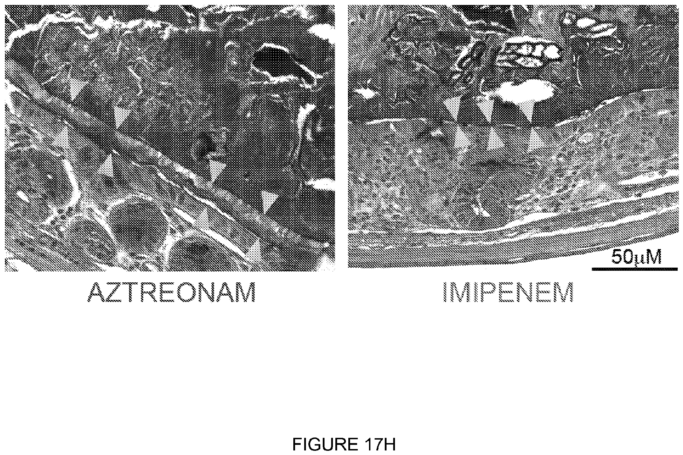

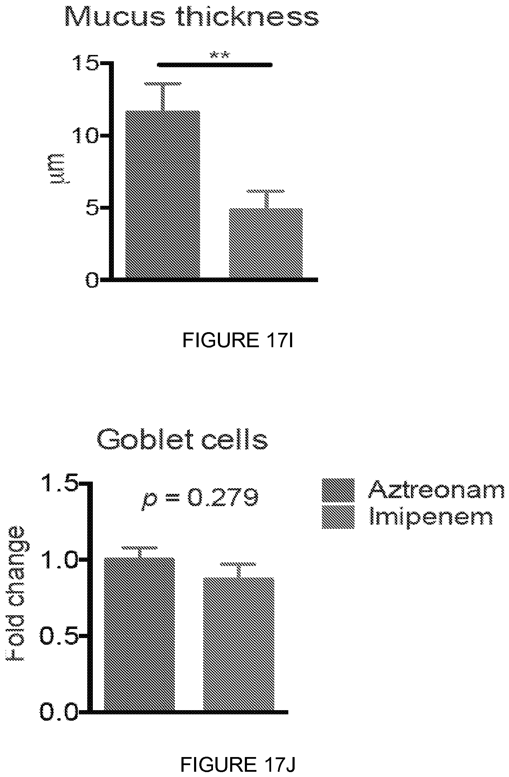

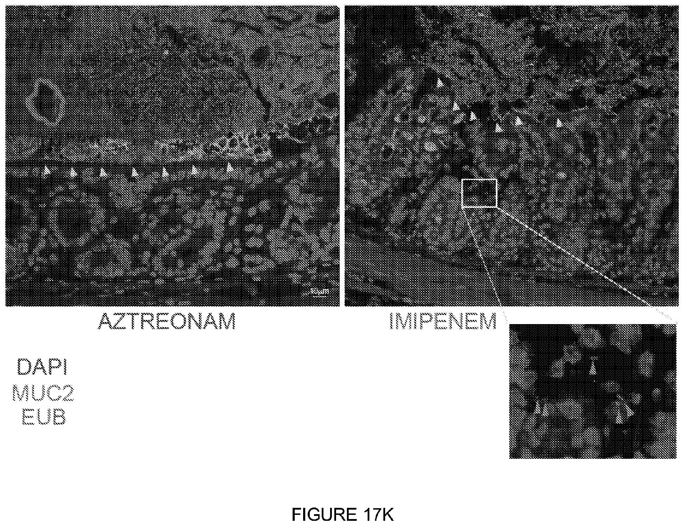

**Figure 17** shows the results of Imipenem treatment after allo-HSCT results in inflammatory and barrier changes in the colon. **(A** to **G)** Lethally irradiated 129S1 recipients were transplanted with C57BL/6 TCD-BM cells with 1 × 10⁶ C57BL/6 T cells. Recipients were treated with imipenem or aztreonam as described in Fig. 2. (A) Colonic lamina propria-infiltrating leukocytes from recipients were analyzed on day 21 by flow cytometry. Data are combined from two independent experiments. Values represent mean ± SEM (n = 10-14). *, P < 0.05; **, P < 0.01. **(B)** Serum, whole small intestine homogenate, and whole colon homogenate levels of IL-23 are shown. Data are combined from two independent experiments. Values represent mean ± SEM (n = 10-12). *, P < 0.05. **(C)** Lamina propria-infiltrating leukocytes from the colon on day 21 were enriched for CD11b and CD11c simultaneously using a mixture of magnetic beads and IL-23 transcripts were quantified by real time PCR. Data are representative of two independent experiments. Values represent mean ± SEM (n = 3). *, P < 0.05. **(D)** Immunofluorescent staining was used to quantify pSTAT3, CD3, and DAPI positive cells in colonic tissue collected on day 21. **(E)** RNA sequencing analysis of the distal colon on day 16 after allo-HSCT. The top 50 regulated genes are shown in the heatmap panel. **(F)** Immunofluorescent staining was used to quantify CD11b, B220, and DAPI-positive cells in colonic tissue collected on day Data are representative of two independent experiments. Values represent mean ± SEM (n = 7-8). **, P < 0.01; ***, P < 0.001; ****; P < 0.0001 in D and F. **(G)** Quantification of gene sequences by homology was performed on stool samples collected on day 21. Amuc_0953, a sulfatase, and Amuc_2164 a glycosyl hydrolase, are two predicted secreted mucolytic genes found in the genome of *Akkermansia muciniphila* ATCC BAA-835, isolated from human feces. **, P < 0.01. **(H** to **J)** Colon tissues from recipients were fixed by water-free Methanol-Carnoy's fixative on day 21, stained with PAS and visualized by light microscopy. Yellow triangles in H indicate the location of the inner mucus layer. Numbers of goblet cells are shown in J. Data are representative of two independent experiments. Values represent mean ± SEM (n = 10). **, P < 0.01. **(K)** Immunostaining for Muc2 (green) of the colon sections with general bacterial 16S rRNA gene FISH probe EUB338 (red) counterstained with Hoechst (blue). Data are representative of two independent experiments (n = 10). Arrowheads; yellow, inner mucus layer; red, bacteria penetrating beyond the mucus layer and colonic epithelium.

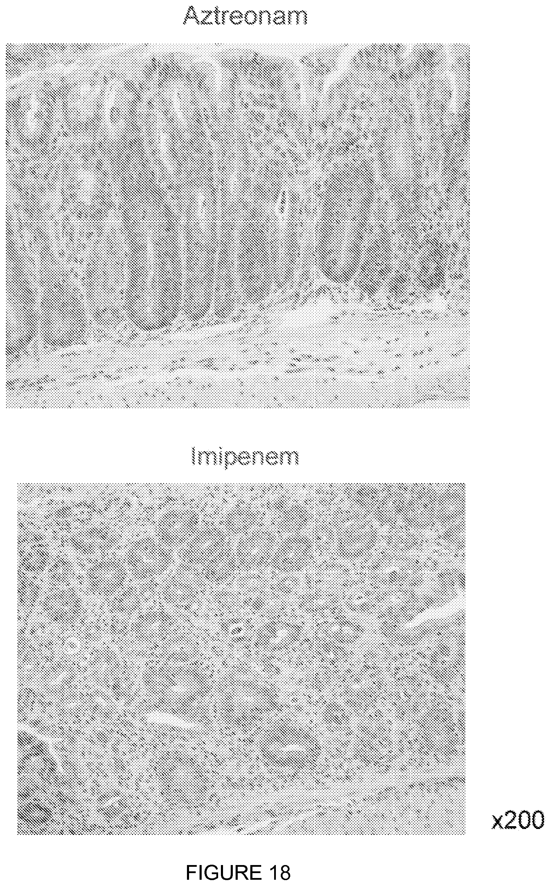

**Figure 18** Recipients treated with imipenem exhibit histological GVHD on day 21. Lethally irradiated 129S1 recipients were transplanted with C57BL/6 TCD-BM cells with 1 × 106 C57BL/6 T cells. Recipients were treated with imipenem or aztreonam (100 mg/kg, SC, 3 times a week from day 10 to day 24). Colon tissues were harvested on day 21. Hematoxylin and eosin stained samples are shown (magnification, ×200). (Left) aztreonam treated recipients; (Right) imipenem treated recipients. Both show evidence of GVHD. The group treated with imipenem exhibits more inflammatory cell infiltration including neutrophils and lymphocytes, robust apoptosis and crypt destruction. Representative histology images from three independent experiments are shown.

### Detailed Description of the Invention

In practicing the present invention, many conventional techniques in molecular biology, microbiology and bacteriology are used, techniques which are within the skill of the art..

With respect to terminology, the terms used herein are intended to be construed in accordance with their standard meaning as known to those of skill in the relevant art. The definition of some terms are given here for convenience.

"Patient" or "subject" as used herein refers to mammals and includes human and veterinary animals.

The terms "intestinal microbiota", "gut flora", and "gastrointestinal microbiota" are used interchangeably to refer to bacteria in the digestive tract.

The term "probiotic" refers to a substantially pure bacteria (i.e., a single isolate), or a mixture of desired bacteria, and may also include any additional components that can be administered to a mammal for restoring microbiota. Such compositions are also referred to herein as a "bacterial inoculant."

The term "prebiotic" refers to an agent that increases the number and/or activity of one or more desired bacteria. Non-limiting examples of prebiotics useful in the methods of the present disclosure include saccharides, such as xylose, raffinose, cellobiose and melizitose.

A "therapeutically effective amount" means the amount of a bacterial inoculant or a compound (e.g., a narrow spectrum antibiotic or anti-bacterial agent) that, when administered to a subject for treating a disorder or condition, is sufficient to effect such treatment.

*"Blautia",* "Blautia-related" or "Blautia-like species" are Gram-stain-positive, non-motile bacteria that are obligate anaerobes found in the feces of humans and other mammals (Liu et al., 2008). *Blautia* species include, for example, *Blautia producta* (ATCC^{R} 27340-DSM 2950, American Type Culture Collection, Manassas, VA). *Blautia-*like species include those with a 16SrDNA sequence with 98% to 100% sequence identity (ifor example 99.5-100% identity) to the 16SrDNA of *Blautia producta* (GenBank X94966). A number of Blautia-related species are shown in Table 1 below by name (NCBI name) including the 16S rDNA sequence of each.

Though not a member of the order *Clostridiales, Holdemania filiformis* is a bacteria associated with less GVHD and therefore is intended to be encompassed by the disclosure as a potential therapeutic.

Antibiotics vary considerably in the strength of their activity against anaerobic commensals and are designated herein as either having high or low activity against anaerobes. Antibiotics with high activity against anaerobes include metronidazole, piperacillin-tazobactam (pip-taxo or P/T) and imipenem. Antibiotics with low activity against anaerobes include aztreonam, ceftazidime/cefepime, iv vancomycin, levofloxacin, ciprofloxacin, cefazolin, atovaquone and tmp-smx.

The relevant taxonomical characteristics of relevant strains of organisms may be confirmed with results obtained from 16S rDNA sequence analysis and the Analytical Profile Index (API^{®}) bacterial identification system in addition to other conventional methods used in the art for bacterial identification.

For patients with hematologic malignancies such as leukemias, lymphomas and other related cancers, allogeneic blood marrow transplantation (allo BMT) or hematopoietic stem cell transplant (HSCT) is a critically important therapy that can produce cures when chemotherapy alone cannot. More than 25,000 patients undergo all BMT worldwide each year. A major risk of bone marrow/hematopoietic stem cell transplant continues to be graft-versus-host-disease (GVHD), which results from the donor immune system recognizing the transplant recipient's organs as foreign, leading to life-threatening inflammation. Developing strategies that reduce GVHD but leave global immune function intact should produce a major benefit for patients.

In the past, the use of broad-spectrum antibiotics in allo-HSCT recipients had been thought to be protective against GVHD. Broad-spectrum combinations were administered with the goal of complete gut decontamination, and this was associated with reduced GVHD in mouse models (36) and in some (37, 38),though not all clinical studies (39-41). Similarly, the addition of metronidazole to ciprofloxacin resulted in reduced GVHD in a small randomized study (42), lending support to the hypothesis that intestinal bacteria contribute to GVHD pathophysiology.

A series of recent studies, however, have described a different association, in which allo-HSCT recipients who sustain more severe microbiota injury are more likely to develop severe GVHD (12, 14, 16, 43). Microbiota injury has been observed in several ways, including expansion of commensal Enterococcus species (12), loss of overall diversity (14), reductions of commensals from the genus Blautia, a member of the order Clostridiales (16), and most recently low levels of indole, a byproduct of tryptophan metabolism produced by intestinal bacteria that can be quantified in the urine in the form of 3 indoxyl sulfate (43). Consistent with these reports, in the current study we demonstrate that use of antibiotics with a broader spectrum of activity, such as imipenem, leads to increased microbiota injury (especially loss of Clostridiales) and increased GVHD severity.

A full explanation has yet to be revealed for the seeming inconsistencies between early studies and more recent studies, but one possible contribution could be the rise of antibiotic-resistant bacteria including resistant enterococci, which can make successful gut decontamination difficult to achieve. Increases in the frequency of colonization with resistant organisms have been observed in allo-HSCT recipients over time (44). A recent study found that gut decontamination was unsuccessful in nearly half of patients where it was attempted. Interestingly, successfully decontaminated patients had a much lower incidence of acute GVHD compared to unsuccessfully decontaminated patients (38).

In this study, we demonstrate that different antibiotics used to treat neutropenic fever have different effects on intestinal microbiota composition, both in patients and in mouse models. We also identified several important changes produced by imipenem treatment in mice with GVHD, including severity of GVHD in the colon, inflammatory changes, and breakdown of the protective colonic mucus barrier.

One promising approach to reducing the risk, incidence and severity of GVHD is targeting the complex community of microbes that reside within the human intestinal tract, collectively termed the intestinal microbiota. While a relationship between the microbiota and GVHD has been suspected for many years, it remains imperfectly understood. Gut decontamination with antibiotics is practiced at some but not all centers, and there is no consensus regarding ideal choice of antibiotic coverage.

Disclosed herein are results demonstrating that the abundance of bacteria belonging to the taxa *Clostridiales* including the genus *Blautia,* a commensal commonly found in the intestinal tract of humans, predicts for protection from life-threatening GVHD in all transplant patients. Furthermore, in murine models, introducing a species of Blautia of murine origin reduces GVHD severity. Not wishing to be bound by theory, it appears to do so by inducing regulatory T cells with generation of short-chain fatty acid metabolic byproducts (SCFA).

Additional studies characterizing the natural history of *Clostridiales* and *Blautia* abundance in all BMT/HSCT recipients demonstrated that the vast majority of patients begin with abundant amounts of endogenous populations of these organisms, but many lose them in a dramatic fashion during the transplantation. Interestingly, loss of *Blautia* correlates strongly with reductions in oral nutritional intake in both humans and mice.

Nutritional intervention strategies to support *Clostridiales*/*Blautia* abundance following all BMT/HSCT may provide one method to mitigate GVHD. It has been shown in murine models that these nutritional approaches can successfully prevent loss of *Clos-tridialeslBlautia* as well as reduce severity of GVHD. These results identified the microbiota as a potent therapeuteic target that can be recruited to significantly reduce GVHD.

The relationship between intestinal microbiota composition and graft-versus-host disease (GVHD) after allo BMT or HSCT is not well understood. Intestinal bacteria have traditionally been thought to contribute to GVHD, but recent animal studies in non-transplant settings have identified populations of obligately anaerobic intestinal commensals with anti-inflammatory properties.

In one study, the fecal bacterial composition of 64 patients was evaluated from day 12 after BMT. Increased bacterial diversity was associated with reduced GVHD-related mortality. Furthermore, harboring increased amounts of bacteria belonging to the genus *Blautia* was associated with reduced GVHD lethality in this cohort and confirmed in another independent cohort of 51 patients. *Blautia* abundance was also associated with improved overall survival. Evaluating the abundance of *Blautia* with respect to clinical factors, it was found that loss of *Blautia* was associated with two clinical factors: 1) treatment with antibiotics that inhibit anaerobic bacteria and 2) receiving total parenteral nutrition (TPN) for longer durations. Increased abundance of commensal bacteria belonging to the *Blautia* genus is associated with reduced lethal GVHD and improved overall survival.

In another study, 283 patients were retrospectively examined for neutropenic fever following allogeneic hematopoietic stem cell transplantation (allo-HSCT). It was found that administering antibiotics with increased activity against anaerobes, including piperacillin-tazobactam (pip/tazo) or imipenem-cilastatin (imipenem), was associated with increased GVHD-related mortality, compared to administering antibiotics, such as aztreonam or cefepime, with reduced activity against anaerobes. Stool microbiota composition analysis demonstrated that pip/tazo and imipenem administration were associated with more severe loss of members of the bacterial order Clostridiales. Experiments in mouse models demonstrated similar flora changes with these antibiotics. Moreover, modeling antibiotic treatment in mice with GVHD recapitulated aggravated mortality with imipenem compared to aztreonam.

The present disclosure describes methods to reduce the likelihood, incidence or severity of, to prevent or otherwise treat GVHD by preventing the loss of or reestablishing certain *Clostridiales* populations in the gut.

IThe disclosure encompasses a method for reducing the risk, incidence or severity of graft versus host disease (GVHD) in a subject undergoing a bone marrow transplant (BMT) or hematopoietic stem cell transplant (HSCT) by taking steps to prevent the loss of beneficial activity, for example, by avoiding the use, when possible of antibiotics that are damaging with respect to anaerobes. The method may comprise selecting/administering to the subject in need thereof an antibiotic with reduced activity for anaerobic bacteria selected from the group consisting of intravenous vancomycin, ceftriaxone, ceftazidime, cefepime, aztreonam, trimethoprim-sulfamethoxazole, ciprofloxacin, levofloxacin and atovaquone.

Restoration of microbiota may be achieved by administering to a subject in need thereof a therapeutically effective amount of a probiotic composition comprising an effective amount of at least one bacterial strain, or a combination of several strains, from the taxa *Clostridiales* wherein the composition (i) stimulates growth and/or activity of bacteria which are protective against GVHD and/or (ii) inhibits growth and/or activity of bacteria which are over-represented in GVHD. Support for protective bacteria can be provided in the form of a nutritional supplement or prebiotic, in some cases, saccharides fermented by the beneficial bacteria. Inhibition of over-represented bacteria, for example, *Akkermansia* by administering an antibiotic that will ablate those organisms and prevent the "crowding out" of the beneficial *Clostridial* species is also contemplated by the disclosure.

The method to reduce the likelihood or severity of GVHD may comprise administering to a subject in need thereof a therapeutically effective amount of a composition comprising one or more bacterial species from the taxa *Clostridiales,* for example, a *Blautia* species/isolate that has been shown to reduce GVHD.

Bacterial strains administered according to the methods of the present disclosure can comprise live bacteria. One or several different bacterial inoculants can be administered simultaneously or sequentially (including administering at different times). Such bacteria can be isolated from microbiota and grown in culture using known techniques.

Administration of a bacterial composition can be accomplished by any method known in the art likely to introduce the organisms into the desired location. The bacteria may be administered orally. Alternatively, bacteria can be administered rectally for example, by enema.

The dosage of the bacterial inoculant or compound of the disclosure will be apparent to the skilled artisan. It is contemplated that a variety of doses will be effective to achieve colonization of the gastrointestinal tract with the desired bacterial inoculant, e.g. 10⁶, 10⁷, 10⁸, 10⁹, and 10¹⁰ CFU for example, can be administered in a single dose. Lower doses can also be effective, e.g., 10⁴, and 10⁵ CFU. Subsequent inoculations, when necessary are envisioned.

Organisms contemplated for administration to restore the gastrointestinal microbiota include those shown in Table 1 below.

### Identification of beneficial species

Species for use in the method described herein may include *Blautia producta* (ATCC^{R} 27340-DSM 2950, American Type Culture Collection, Manassas, VA) or those indicated by an asterisk in Table 1 below. New *Blautia* isolates are being identified every year; in some instances isolates of other genuses are being recategorized as *Blautia.* So, for example, *Blautia*-like or *Blautia*-related species may include *Ruminococcus obeum, Ruminococcus faecis; Ruminococcus lactaris,* etc. (see Table 1 below)

To assess the association with GVHD-related mortality (or any other outcome), a script was utilized that calculates the association of the log-transformed abundance of each bacteria with the time to event of the outcome of interest, using a Cox proportional hazards test. This has the advantage of taking into consideration the time that passes before the event for each patient. The other major advantage is that the Cox proportional hazards test result can be readily adjusted to account for effects of other potential clinical factors that could confound. In this case, we performed the univariate analysis, as well as a multivariate analysis adjusting for type of transplant (cord blood vs. peripheral blood vs. bone marrow) and conditioning intensity.

Data was analyzed at the operational taxonomic unit (OTU) level. The nucleotide sequence information for each specific 16S rRNA was BLASTed against the NCBI 16S database to give the names.

In general, *Blautia* and *Blautia*-related species (including species from the genuses, *Ruminococcus, Lactococcus, Anaerostipes, Holdemania,* and) are Gram-stain-positive, non-motile bacteria that are obligate anaerobes found in the feces of humans and other mammals (Liu et al., 2008). Bacteria shown to have an association with GVHD, whether beneficial or detrimental are shown in Table 1 with those associated with lower risk or incidence of GVHD indicated by *.

**Table 1**

| taxon | p.value_log | HR_log | p.value_log_ source_ intensity | HR log_ source intensity | NCBI.unique_name | SEQ ID NO: |
|---|---|---|---|---|---|---|
| OTU_113;size=47975 | 0.085279424 | 0.91545478 | 0.04611402 | 0.903106 | [Ruminococcus]_obeum* | 1 |
| OTU_1645;size=734 | 0.072004042 | 1.08173779 | 0.08050389 | 1.081424 | Bifidobacterium_dentium | 2 |
| OTU_1982;size=64 | 0.048192219 | 0.84281324 | 0.0387293 | 0.836018 | [Clostridium]_hathewayi* | 3 |
| OTU_226;size=6379 | 0.186963794 | 0.92465591 | 0.08036057 | 0.899248 | Eubacterium_desmolans* | 4 |
| OTU_33;size=130106 | 0.105709271 | 0.93046481 | 0.05978686 | 0.921585 | Dorea_longicatena* | 5 |
| OTU_371;size=1222 | 0.137347553 | 1.08413436 | 0.07894683 | 1.103009 | Lactococcus _ raffinolactis | 6 |
| OTU_376;size=6163 | 0.111144131 | 0.89194849 | 0.04725679 | 0.86433 | Ruminococcus_lactaris* | 7 |
| OTU_634;size=499 | 0.071602537 | 0.83912625 | 0.06876381 | 0.835283 | [Eubacterium]_contortum* | 8 |
| OTU_64;size=60078 | 0.063469937 | 0.91183353 | 0.05563582 | 0.908939 | Ruminococcus_faecis* | 9 |
| OTU_701;size=696 | 0.106962035 | 1.09834607 | 0.04763833 | 1.125336 | Lactobacillus_parafar-raginis | 10 |
| OTU_76;size=93266 | 0.102147698 | 1.08177518 | 0.05777609 | 1.095514 | Lactobacillus_reuteri | 11 |
| OTU_77;size=44540 | 0.099796012 | 0.91161177 | 0.04365446 | 0.894198 | Holdemania_filiformis* | 12 |
| OTU_82;size=29188 | 0.050020607 | 1.08837291 | 0.0787453 | 1.078644 | Acidaminococcus_intestini | 13 |
| OTU_880;size=16378 | 0.009699651 | 1.11118429 | 0.00577229 | 1.111824 | [Eubacterium]_biforme | 14 |
| OTU_955;size=4201 | 0.095422614 | 0.89097705 | 0.0551945 | 0.874574 | [Clostridium]_sordellii* | 15 |
| OTU_983;size=864 | 0.142891624 | 1.14855657 | 0.08220527 | 1.179152 | Serratia_fonticola | 16 |

| | | | | | | |
|---|---|---|---|---|---|---|
| * Associated with lower risk or incidence of GVHD | | | | | | |

*Blautia* and *Blautia*-like species, particularly those strains with a 16SrRNA sequence that closely matches that of the 16SrRNA sequence (GenBank X94966) of *Blautia producta* or any of SEQ ID NOS: 1, 3, 4, 5, 7, 8, 9, 12 and 15 (for example, with 98% to 100% sequence identity or 99.5-100% identity) are suitable for use in the disclosed methods.

The abundance of bacteria from the taxa *Clostridiales,* which includes *Blautia,* has also been shown to be predictive for reduced GVHD-related mortality in some patients. Interestingly, several of 17 clostridial isolates tested (for characterization, see Narushima et al. Characterization of the 17 strains of regulatory T cell-inducing human-derived Clostridia. Gut Microbes 5:3, 333-339 2014) are very close relatives and therefore, may be useful in practicing the method disclosed herein.

Methods for determining whether a *Blautia* or Blautia-like species or isolate is suitable for use in the present disclosure may include determining the percent identity of the 16SrRNA of a species/isolate with the 16SrRNA sequence of *Blautia producta* (GenBank X94966) or any of SEQ ID NOS: 1-16; methods for doing so are well known in the art.

*Blautia* species for use in the present disclosure include those *Blautia* and *Blau*tia-like species that ferment certain sugars, for example, xylose, raffinose, cellobiose and melizitose. Provision of nutritional supplements comprising these sugars may be suitable for administration to a subject as a prebiotic strategy for reducing GVHD.

### Administration of BlautialBlautia-related species

One or more different bacterial inoculants can be administered simultaneously or sequentially (including administering at different times). Bacterial strains administered according to the methods of the present disclosure can comprise live bacteria, frozen bacteria, bacterial spores or a combination thereof. Such bacteria can be isolated from an appropriate microbiota source or obtained from a cell repository (such as the American Type Culture Collection/ATCC) and grown in culture using known techniques.

Delivery of *Blautia* species to a subject to reduce the likelihood, incidence, severity or otherwise prevent or treat GVHD may be accomplished using any oral delivery system suitable for administering live microorganisms to an individual in need thereof, for example, as described in U.S. published application no. 20140112985. Such a delivery system may comprise a probiotic agent that comprises at least one species of live *Blautia* microorganisms that have been shown to correlate with reduced GVHD; optionally, at least one additional agent for example, intestinal motility agents; and a delivery vehicle, wherein the oral delivery vehicle releases the probiotic agent to the distal small intestine of the individual. A *Blautia* isolate may be administered in combination with a sugar that it is known to ferment.

Oral delivery may be achieved via a vehicle selected from the group consisting of pills, tablets, caplets, capsules, soft gels, and coated probiotic granules, that will release the probiotic agent in the distal small intestine. The disclosure further provides for oral delivery wherein the probiotic agent is present, and is in a dosage form selected from immediate release, delayed release, extended release which is released in the distal small intestine, and targeted release which is targeted to be released in the distal small intestine.

### EXAMPLES

### Selection of patients and method for specimen collection

In one study, subjects analyzed retrospectively for impact of antibiotics on clinical outcomes consisted of 283 adult patients undergoing all-HSCT at Memorial Sloan Kettering Cancer Center (MSKCC) from 1994 to 2013. Patients who received conventional grafts (non-T cell depleted) were included in this study; patients who received ex-vivo T cell depleted grafts or peri-transplant alemtuzumab were excluded. Stool specimens were collected and stored weekly over the course of the transplant hospitalization. The study was approved by the Institutional Review Board at MSKCC. All study patients provided written informed consent for biospecimen collection and analysis.

GVHD was diagnosed clinically, confirmed pathologically by biopsy whenever possible, and classified according to historical consensus criteria as described previously (see Rowlings PA, Przepiorka D, Klein JP, et al. IBMTR Severity Index for grading acute graft-versus host disease: retrospective comparison with Glucksberg grade. Br J Haematol. 1997). These criteria were applied to GVHD with purely acute features that occurred after day 100. Cases of GVHD were further categorized by treatment with or without systemic steroids (prednisone or methylprednisolone, 0.5 mg/kg daily or higher). Cause of death was determined using a standard algorithm where outcomes were prioritized in the following order: 1) primary disease recurrence, 2) graft failure, 3) GVHD, 4) infection, and 5) organ failure; thus in patients without disease recurrence or graft failure, those who were being treated for GVHD at the time of death were considered to have succumbed to GVHD-related mortality, including those who died from infections. Disease risk was determined according to the ASBMT RFI 2014 Disease Classification. Conditioning intensity was assigned based on previously established working definitions.

### Sample storage and DNA extraction

Stool samples from patients were stored at 4°C for <24 h before freezing at - 80°C. Ileal and large intestinal samples from mice were frozen at -80°C. DNA was extracted using one of two methods, which give similar results.

For each stool specimen, DNA was extracted using a phenol-chloroform extraction technique (see Ubeda, C., Y. Taur, R.R. Jenq, M.J. Equinda, T. Son, M. Sam-stein,A. Viale, N.D. Socci, M.R.M. van den Brink, M. Kamboj, and E.G. Pamer. 2010. Intestinal domination by Vancomycin-resistant Enterococcus precedes bloodstream invasion in humans. J. Clin. Invest.) or using Power Soil DNA isolation kit (MO BIO Laboratories).

### 16S analysis of specimen

For each stool specimen, DNA was extracted and purified. Samples were analyzed using the 454 GS FLX Titanium platform to sequence the V1-V3 region of the bacterial 16S rRNA gene or were alternatively analyzed using the Illumina MiSeq platform to sequence the V4-V5 region of the 16S rRNA gene. Sequence data were compiled and processed using mothur version 1.34 and QIIME version 1.8.0, screened and filtered for quality. Operational taxonomic units (OTUs) were classified to the species level using a modified form of the Greengenes reference database. Principal component analysis was performed upon a weighted and normalized Unifrac distance matrix of OUT abundance in R software. Data from this study has been stored in the NCBI Sequence Read Archive (url: ncbi.nlm.nih.bov/sra).

Phylogenetic abundance comparisons were performed in order to identify biomarkers of GVHD-related mortality using linear discriminant analysis (LDA) effect size (LEfSe) analysis27, using a logarithmic LDA cutoff of 2.0.

### Antibodies and Flow Cytometry

All antibodies were obtained from BD Biosciences-Pharmingen. For cell analysis of surface markers, cells were stained for 20 minutes at 4°C in PBS with 0.5% BSA (PBS/BSA) after Fc block, washed, and resuspended in DAPI in PBS/BSA. Cell surface staining was followed by intracellular staining with the eBioscience kit per the manufacturer's instructions. Dead cells were excluded with LIVE/DEAD Fixable Dead Cell Stain kit (Invitrogen). All flow cytometry was performed on an LSR II (BD Biosciences) and analyzed with FlowJo (TreeStar Software).

### Antibiotics Categories

Antibiotics used during transplant hospitalization were divided into those that included significant activity against anaerobic bacteria (pip/tazo, ticarcillin clavulanate, imipenem, meropenem, metronidazole, oral vancomycin and clindamycin), and those with reduced activity (intravenous vancomycin, ceftriaxone, ceftazidime, cefepime, aztreonam, trimethoprim-sulfamethoxazole, ciprofloxacin, levofloxacin, atovaquone (19).

### Transplantation Practices

As per our institutional practice, patients received ciprofloxacin prophylaxis, and those undergoing more intense conditioning than nonmyeloablative regimens also received intravenous vancomycin prophylaxis starting day -2 through day 7 (59). Antibiotic prophylaxis against Pneumocystis jiroveci (trimethoprim sulfamethoxazole, aerosolized pentamadine, or atovaquone) was given at the discretion of the transplant physician.

### Analysis of specimens

For each stool specimen, DNA was purified using a phenol-chloroform extraction technique with mechanical disruption (bead-beating) based on a previously described protocol (60). Samples were analyzed using the 454 GS FLX Titanium platform to sequence the V1-V3 region of the bacterial 16S rRNA gene or were alternatively analyzed using the Illumina MiSeq platform to sequence the V4-V5 region of the 16S rRNA gene. Sequence data were compiled and processed using mothur version 1.34 (61), screened and filtered for quality (62). Operational taxonomic units (OTUs) were classified to the species level (63) using a modified form of the Greengenes reference database (64). Principal component analysis was performed upon a weighted and normalized Unifrac (65) distance matrix of OTU abundance in R software. Data from this study has been stored in the NCBI Sequence Read Archive (see url: www.ncbi.nlm.nih.gov/sra).

### RNA Sequencing and Analysis

Mice were sacrificed on day 16 after receiving a total of three times of antibiotics treatment. The distal colon was removed and pooled (n = 4; both for aztreonam and imipenem treatement groups), followed by RNA isolation using TrizolLS. RNA was prepared using RiboMinus (LifeTechnologies). The library was sequenced using the Ion Proton System (LifeTechnologies). Aligned RNA was analyzed for fold change. Differential gene expression was assessed in imipenem vs. aztreonam treated mice.

### Metagenomic Shotgun Sequencing and Analysis

Paired-end raw reads from shotgun sequencing were trimmed using Trimmo-matic 0.32 (69) using a maximum mismatch of 2, minimum terminal base score of 30, and the Illumina TruSeq adapter sequences. The remaining clipped reads were taxonomically assigned using Kraken (70). Briefly, trimmed and filtered reads were taxonomically classified by k-mer resemblance to bacterial, viral and fungal k-mer profiles generated from the NCBI Genome and Chromosome collections (accessed November 12, 2014). Unclassified reads were further interrogated with BLAST (nt database, March 24, 2015) and non-microbial reads were discarded. Functional analysis was conducted on quality filtered reads using HUMAnN v0.99 (71), which determines the abundance of genes and pathways in a given metagenomic community. To identify those functional categories that were differentially represented between the aztreonam and imipenem-treated subject samples, we employed LEfSe (21); a validated tool that identifies differentially abundant biomarkers such as genes, pathways or organisms between microbial communities.

Recipients were sacrificed on day 21 and 10 mm long segments of colon together with fecal content were carefully collected and soaked into a water-free Methanol-Carnoy's fixative (60% dry methanol, 30% chloroform and 10% acetic acid) (72) overnight. The tissues were then washed in methanol, embedded in paraffin, and then 5 µm sections were placed on glass slides. Slides were deparaffinized, and stained with standard Periodic acid-Schiff method, and assessed by light microscope (73).

### Immunostaining and fluorescence in situ hybridization (FISH) of the colon tissues

Formalin-fixed colons from recipients were stained with anti-mouse CD3 antibody A0452 (DAKO), pSTAT3 antibody 9135 (Cell Signaling), CD11b antibody ab133357 (Abcam), B220 antibody 550286 (BD Pharmingen), versus isotype control. Immunofluorescence secondary staining was performed with AF488 for pSTAT3 and B220, and AF594 for CD3 and CD11b. Pieces of colon with fecal material were fixed in Carnoy and bacterial FISH (EUB338) (35) and immunostainings were done with MUC2C3 antiserum and DNA by Hoechst 34580 (Life technologies) as previously described (74, 75).

### Mice and bone marrow transplantation and assessment of graft versus host disease.

Female C57BL/6, C57BL/6/Ly5.1, and 129S1/SvlmJ mice were obtained from the Jackson Laboratory (Bar Harbor, Maine, USA). Mice used for experiments were 6-9 weeks old. Mice were treated with a gut-decontaminating antibiotic cocktail (ampicillin and vancomycin) to mimic microbiota injury that occurs in allo BMT patients. Mice were then exposed to a myeloablative dose of total body irradiation (TBI, 11 Gy from a 137Cs source as a split dose with a 3-hour interval between doses) and then transplanted by intravenous injection with bone marrow and purified splenic T cells from fully MHC-mismatched B10.BR mice (H2k into H2b). Donor mice were euthanized by asphyxiation using carbon dioxide, and spleens, femurs, and tibias were removed aseptically. Donor BM was obtained by flushing of tibia and femora with cold tissue culture media. Donor BM was T cell depleted (TCD) by incubation with 2.5 µg anti-Thy-1.2 per 106 BM cells for 30 minutes at 4°C, followed by incubation with 10 µL of low-TOX-M rabbit complement per 10⁶ BM cells for 40 minutes at 37°C, so that GVHD could be reproducibly induced by simultaneous injection of T cell-depleted BM and donor splenic T cells in experimental mice. Splenic T cells were purified with anti-CD5 MACS beads (Miltenyi). The BM cells (5 × 10⁶ per recipient) and splenic T cells (1 × 106 per recipient) were transplanted by tail vein injection.

### Isolation of Blautia isolates from mouse or human feces

Entire stool specimens are collected and homogenized in 1-3 volumes of 0.05% peptone using a sterile stainless steel blender with 1-3 volumes of peptone. Approximately 1 gram of the specimen is serially diluted (10-fold) in pre-reduced, anaerobically sterilized (PRAS) dilution blanks (Anaerobe Systems). A separate ~1 gram aliquot is weighed, dried in a vacuum overnight, and re-weighed in order to calculate counts on a dry-weight basis. To select for Clostridiales bacteria, including *Blautia* species, 100 µL of the homogenized stool sample dilution series is plated on Brain-Heart Infusion blood agar (SBA, Becton Dickinson) supplemented with 4 µg/mL trimethoprim (Sigma Chemical) and 1 µg/mL sulfamethoxazole (Sigma), Brucella Blood Agar (BAP, Anaeobe Systems), CDC ANA blood agar, (BBL Microbiology Systems), and egg yolk agar (EYA, Anaerobe Systems) (Finegold SM, Molitoris D, Song Y, Liu C, Vaisanen ML, Bolte E, McTeague M, Sandler R, Wexler H, Marlowe EM, Collins MD, Lawson PA, Summanen P, Baysallar M, Tomzynski TJ, Read E, Johnson E, Rolfe R, Nasir P, Shah H, Haake DA, Manning P, Kaul A, 2002. Gastrointestinal microflora studies in late-onset autism. Clin Infect Dis 1:35). To select for spore-formers, the dilutions may be heated at 70-80°C for 10-20 minutes and plated in the same manner as the non-heated homogenized stool samples. After 5 days of growth at 37°C in an anaerobic chamber, single colonies are selected. The colony purification process is repeated by restreaking select single colonies, growing as described above, and selecting again for single colonies. Single colonies are frozen in 15%-25% glycerol in 1 mL cryotubes and stored at -80°C.

### Administration of Blautia/consortia to mitigate experimental GVHD

C57BL/6 mice purchased from The Jackson Laboratory (Bar Harbor, Maine) were treated with oral vancomycin and ampicillin. Following decontamination, mice were housed in autoclaved conditions (caging, bedding, water and food) to eliminate nearly all endogenous Clostridia present within the flora of mice. Mice were then treated by gavage with either a liquid suspension of cultured *Enterococcus faecalis* as a control, or a *Blautia* isolate. Mice were then exposed to a myeloablative dose of total body irradiation (TBI, 11 Gy) and then transplanted by intravenous injection with bone marrow and purified T cells from fully MHC-mismatched B10.BR mice (H2k into H2b). Effects on intestinal pathology and overall survival were evaluated as described. Mice colonized by *Blautia,* compared to those harboring Enterococcus, were protected from GVHD, with improved survival **(Figure 9).**

### GVHD clinical and histological scoring

Mice were monitored daily for survival and weekly for GVHD clinical scores (see Cooke, K.R., L. Kobzik, T.R. Martin, J. Brewer, J. Delmonte Jr.,J.M. Crawford, and J.L. Ferrara. 1996. An experimental model of idiopathic pneumonia syndrome after bone marrow transplantation: I. The roles of minor H antigens and endotoxin. Blood. 88:3230-3239). Small intestine, large intestine, and liver samples were evaluated histologically for evidence of GVHD and scored as previously described (see Hill, G.R., J.M. Crawford, K.R. Cooke, Y.S. Brinson, L. Pan, and J.L. Ferrara. 1997. Total body irradiation and acute graft-versus-host disease: the role of gastrointestinal damage and inflammatory cyto-kines. Blood.90:3204-3213).

### Measurement of paneth cell numbers and functionality

The small intestinal lumens of adult mice are rinsed with ice-cold water and segmented. Crypts are eluted by first turning the segments inside out and then shaking them in PBS containing 30 mM EDTA and lacking Ca2+ and Mg2+. The eluted villi and crypts are pelleted at 700xg, resuspended in PBS, and transferred to siliconized microfuge tubes using capillary pipettes. The crypts are resuspended in iPIPES buffer (10 mM PIPES (pH 7.4) and 137 mM NaCl) in preparation of exposure to secretory stimuli.

Crypts are incubated in 30 µl of iPIPES containing 1000 bacterial (Clostridiales) CFU per crypt for 30 min at 37 °C. Cellular components are pelleted by brief centrifugation, and supernatants transferred to sterile microfuge tubes and stored at -20 °C. This method may be scaled up using up to ~3000 crypts in 2 ml iPIPES (plus or minus Clostridiales bacteria). Crypts are pelleted and 10 µL of the supernatants are analyzed for bactericidal activity against Clostridiales and Enterococcus bacteria in liquid culture or on agar plates. Proteins are extracted from the rest of the supernatant as well as the crypts using 30% acetic acid. Total protein extracted from each fraction was resolved by AU-PAGE and subjected to western blot analysis using anti-cryptdin-1 as follows. Proteins from AU-PAGE are transferred to a nitrocellulose membrane. The membrane is then blocked with 5% skim milk, incubated sequentially with anti-rabbit mouse cryptdin-1 (1:500), horseradish peroxidase-conjugated anti-rabbit IgG (1:20,000) and chemilumi-nescent substrate (SuperSignal, Pierce, Rockland, IL), and visualized (Ayabe T, Satchell DP, Wilson CL, Parks WC, Selsted ME, Ouellette AJ, 2000. Secretion of microbicidal α-defensins by intestinal Paneth cells in response to bacteria. Nature Immunology 1:113-118).

### Method for measuring levels of microbes in distal organs (liver, thymus, lungs, kidneys)

Quantitative PCR (qPCR) of bacterial 16S rRNA genes was performed on tissue samples using DyNAmo SYBR Green qPCR kit (Finnzymes) and 0.2 µM of the universal bacterial primer 8F (5'-AGAGTTTGATCCTGGCTCAG-3' SEQ ID NO: 1) and the broad-range bacterial primer 338R (5'-TGCTGCCTCCCGTAGGAGT-3' SEQ ID NO: 2). Standard curves were prepared by serial dilution of the PCR blunt vector (Invitrogen) containing 1 copy of the 16s rRNA gene.

### Method for measuring effect of microbes on bacterial metabolites such as SCFA levels.

Short-chain fatty acids (SCFA) are produced by many bacteria as a byproduct of carbohydrate fermentation. SCFA have been found to be important modulators of the immune system. They are abundantly produced by *Blautia* and related bacteria from the Class Clostridia. To evaluate how *Blautia* and related bacteria mediate suppression of GVHD, fecal pellets were collected to quantify SCFA levels, particularly acetate, propionate, or butyrate. SCFAs, creatines, and hydroxy-SCFAs were quantified by alkalinizing stool samples, obtaining fingerprints of the metabolic composition of the sample using 1D 1H NMR on a Bruker Avance-600 MHz Spectrometer, and analyzing with supervised multivariate statistical methods using Chenomx NMR Suite software.

### Administration of SCFA to mitigate GVHD.

Preliminary experiments were done to test the impact of administration of SCFA on murine GVHD. A significant benefit of propionate given via drinking water (data not shown), or butyrate given via drinking water or via enema (data not shown) was not observed while a notable benefit with administration of acetate via drinking water was. Sodium acetate (150mM) will be delivered via the drinking water of mice beginning 2 weeks prior to BMT. Mice will then be irradiated and transplanted with continued supplementation of sodium acetate. Outcomes that will be evaluated in mice include GVHD clinical scores, survival, and day 14 and 28 tissue pathology. Kaplan-Meier curves will display the overall survival for the two groups. In addition, the area under the curve (AUC) will summarize the weekly total GVHD score from the time of infusion to week 13 for each mouse. Mice will be euthanized to evaluate for pathological evidence of GVHD, as well as to quantify and characterize large intestinal Tregs and alloreactive effector T cells by flow cytometry on days 14 and 28.

### Method for measuring effect of microbial metabolites (SCFA) on intestinal crypt regeneration

An intestinal epithelial crypt culture system as previously described (Toshiro Sato, Robert G. Vries, Hugo J. Snippert, Marc van de Wetering, Nick Barker, Daniel E. Stange, Johan H. van Es, Arie Abo, Pekka Kujala, Peter J. Peters & Hans Clevers, 2009) was used. Single Lgr5 stem cells build crypt-villus structures in vitro without a mesenchy-mal niche, Nature, 459:262-265). 400 crypts per well were suspended in liquefied growth factor reduced Matrigel (Corning) (25% Advanced DMEM / F12 medium (Gibco); 75% growth factor reduced Matrigel) at 4°C. Then, they were plated in pre-warmed delta-surface Nunc 24-well plates in 50 µL drops for small intestine, 30ul drops for large intestine, each containing approximately 100-500 crypts. After the Matrigel drops polymerized, 500ul complete crypt culture medium was added to small intestine crypt cultures (ENR-medium: advanced DMEM/F12 (Sigma), 2mM L-glutamine (Sigma), 10mM HEPES (Sigma), 100U/ml penicillin/100µg/ml streptomycin (Sigma), 1mM N-acetyl cysteine (Sigma), 1 × B27 supplement (Invitrogen,), 1 × N2 supplement (Invitrogen), 50ng/ml mEGF (Peprotech), 100ng/ml mNoggin (Peprotech) and 10% human R-spondin-1 conditioned medium of hR-spondin-1-transfected HEK 293T cells. In some experiments evaluating budding hR-spondin-1 was lowered to 1.25-5%. Large intestine crypts were cultured in WENR-medium containing 50% of Wnt3a conditioned medium in addition to the aforementioned proteins and 1% Bovine serum Albumin (Sigma). For large intestine cultures 10uM SB202190 (Sigma, Cat.nr.S7067) and ALK5 inhibitor (A83-01, Tocris) were added to the WENR. All plates were incubated at 37 °C/5% CO2 and the media was replaced every 2-3 days. Control wells were left untreated, and where applicable, treatment wells received different concentrations of bacterial metabolites along with medium changes. Crypts were passaged at day 7 by mechanically disrupting them with a seropipet, washing away the Matrigel by spinning down the crypts in excess medium, and replating them after reconstitution of the pellet in liquefied Matrigel.

### Selection of oligosaccharides to augment Blautia

Utilizing *Blautia* isolate of C57BL/6 origin, as well as a *Lactobacillus johnsonii* of C57BL/6 origin, the ability of these bacteria to ferment a variety of sugars was evaluated using pH and optical density to evaluate bacterial growth in media lacking glucose. *Lactobacillus johnsonii* was evaluated because this bacteria expands in the setting of calorie restriction at the expense of Clostridia, and is thus presumably a direct competitor for nutrients in the murine intestine. Two sugars that are fermented by *Blautia* and not by *Lactobacillus:* rhamnose and xylose were identified from this analysis.

### Administration of oligosaccharides to augment Blautia and mitigate experimental GVHD

C57BL/6 mice were inoculated orally with a murine Blautia isolate known to ferment xylose. Some mice were then administered xylose in the drinking water (10g/L) beginning 7 days prior to BMT with B10.BR BM and T cells. These mice receiving xylose exhibited an expansion of *Blautia,* measured by 16S deep sequencing, in the intestinal flora despite the presence of GVHD on day 14 after BMT **(****Figure 9****).** Interestingly, long-term administration of xylose also led to improved survival of mice with GVHD.

A further study shows the growth of strains from a clostridial mix, in BHI media without glucose to which various sugars are added. Glucose gave the best results and was able to support the growth of 10 of the total 17 strains. Glucose, however would have limited benefit since it likely doesn't give a selective advantage to the beneficial bacteria. Raffinose was able to support 5 strains, while cellobiose appeared to support 4 strains not supported by raffinose. A mixture of raffinose and cellobiose can be administered to a subject to provide support for at least a portion of the beneficial bacteria.

### Detection of metabolites produced by cultured Blautia.

Bacterial metabolites including SCFAs, creatines, and hydroxy-SCFAs will be quantified by obtaining fingerprints of the metabolic composition of the sample using 1D 1H NMR on a Bruker Avance-600 MHz Spectrometer, and analyzing with supervised multivariate statistical methods using Chenomx NMR Suite software.

### In vitro assay to demonstrate suppression of Blautia by dominant dysbiotic microbes due to free radical production

From studying the effects of calorie restriction on intestinal microbiota composition it was observed that obligate anaerobes (Clostridia, Bacteroidetes) are reduced in abundance while facultative anaerobes (Lactobacillales, Proteobacteria) expand. Production of free radicals by *E. coli* in the setting of starvation has been described previously (Saby S, et al. Appl Environ Microbiol. 1999; 5600-5603.). Experiments were designed to examine in vitro if Lactobacillus johnsonii could suppress the growth of our murine *Blautia* isolate under starvation conditions, and to further investigate if production of free radicals could be a contributing factor. Specifically, *Blautia* was cultured either alone, or with *L*. *johnsonii,* and it was observed that *L. johnsonii* indeed suppressed the growth of *Blautia,* but failed to do so if additional media was added to prevent starvation **(****Figure 11****).** The effects of media that had supported the growth of *L. johnsonii* to plateau phase were further investigated following sterile filtration. Lacto-conditioned media had no effect on the growth of *Lactobacillus* when mixed with fresh media in a 1:1 ratio, but could suppress the growth of *Blautia,* indicating that under starvation conditions, *Lactobacillus* releases substances that suppress the growth of *Blautia* but not itself. It was then examined if reducing agents could rescue *Blautia* from Lacto-conditioned-media-mediated suppression. L-cysteine, ascorbic acid, and sodium thioglycolate were all found to be able to support *Blautia* growth in the presence of Lacto-conditioned media. Together, these results suggest that *Lactobacillus* may develop a competitive advantage over *Blautia* in the setting of limited nutrients due to production of free radicals, which are selectively damaging to obligately anaerobic bacteria due to a lack of protective enzymes including glutathione transferases, catalases, and superoxide dismutases.

### In vitro combination of Blautia + xylose and effects on bacterial metabolites

*Blautia* was cultured in liquid PY medium alone, or supplemented with glucose or xylose (10g/L), for 48 hours. Media was centrifuged and the supernatant was evaluated for levels of SCFA.

### Antibiotic categories

Antibiotics used during transplant hospitalization were divided into those that included significant activity against anaerobic bacteria (piperacillin-tazobactam, ticarcillin-clavulanate, imipenem-cilastatin, meropenem, metronidazole, oral vancomycin and clindamycin), and those with reduced activity (intravenous vancomycin, ceftriaxone, ceftazidime, cefepime, aztreonam, trimethoprim-sulfamethoxazole)¹⁹.

### Transplantation practices

As per institutional practice, patients received ciprofloxacin prophylaxis, and those undergoing conditioning more intense then nonmyeloablative regimens also received intravenous vancomycin prophylaxis starting day -2 through day 7²⁰. Antibiotic prophylaxis against *Pneumocystis jiroveci* (trimethoprim-sulfamethoxazole, aerosolized pentamadine, or atovaquone) was given at the discretion of the transplant physician.

### Statistical analysis

The incidence of acute GVHD and GVHD-related mortality was estimated using cumulative incidence functions, treating relapse and death unrelated to GVHD as competing events, and compared across factors using Gray's test. Overall survival probabilities were estimated using Kaplan-Meier methodology and compared using the logrank test. Comparisons of bacterial abundance were performed using the Mann-Whitney U for unpaired tests. For mouse experiments, data were presented as mean ± SEM. Survival curves were analyzed with the Mantel-Cox log-rank test. For other comparisons, nonpar-ametric Mann-Whitney U test was used. In all analyses statistical significance was defined as P < 0.05 based on a 2-sided test. Statistical analyses were performed using R version 3.1.0 (The R Foundation for Statistical Computing, Vienna, Austria) and GraphPad Prism version 6.00 for Machintosh, (GraphPad Software, San Diego, California, USA).

### Composition of the intestinal flora and GVHD-related mortality - impact of diversity and identifying predictive subsets of bacteria

Our group recently reported that increased bacterial diversity at the time of engraftment was associated with improved overall survival following allo BMT and reduced transplant-related mortality, but in that heterogeneous patient population we were unable to determine if diversity was associated with reduced GVHD¹³. For the current study, we began by asking if bacterial flora diversity could predict for lethal GVHD in a more uniform population of patients at high risk for developing GVHD. We utilized banked stool samples collected from patients who underwent allo BMT at our center. We identified a cohort of 64 patients who, following conventional allo BMT without T cell depletion, had provided a stool sample following BMT infusion and prior to hospital discharge (collected day 8-16, median day 12; clinical characteristics summarized in Table 2). We analyzed the flora composition of these stool samples by sequencing regions V1-V3 of the 16S rRNA gene using the 454 platform, and followed patients clinically for development of GVHD-related mortality.

**Table 2**

| Clinical characteristics of 64 allo BMT patients transplanted at MSKCC with stool samples collected day +12 after BMT included in an identification cohort. | |
|---|---|
| Dates of transplant | September 2009 to October 2012 |
| Age (years) | 25 to 70, median 53 |
| Gender | Female 38%, male 62% |
| Primary malignancy | NHL 38%, AML 38%, ALL 9.4%, Hodgkin disease 6.3%, CLL 6.3%, MDS 3.1% |
| Disease risk | High 45%, intermediate 36%, low 19% |
| Graft source | Peripheral blood 55%, cord blood 42%, bone marrow 3.1% |
| Donor relationship/HLA | Sibling identical (29%), unrelated identical (22%), unrelated non-identical (48%) |
| Conditioning intensity | standard intensity myeloablative 23%, reduced intensity myeloablative 44%, nonmyeloablative 33% |
| Stool sample collection day | +8 to +16, median +12 |

We ranked patients by the median Shannon diversity index into two equal groups and found that increased bacterial diversity was indeed associated with reduced GVHD lethality **(****Figure 1A****,** p=0.005). To identify bacterial subsets associated with either increased or decreased GVHD-related mortality, we compared the abundances of bacterial genera from patients who did or did not die from GVHD by linear discriminant analysis (LDA) effect size (LEfSe)²⁷ as a hypothesis-generating approach. We found that bacteria belonging to the genus *Blautia* were most significantly associated with reduced GVHD-related mortality **(****Figure 1B****,** p=0.01). The *Blautia* genus notably includes anaerobic intestinal commensal organisms within the bacterial class Clostridia^{28,29}.

We evaluated *Blautia* abundance as a predictive factor for GVHD-related mortality, stratifying patients by the median *Blautia* abundance of 0.05%, and found that a higher abundance of *Blautia* was associated with reduced GVHD-related mortality (Figure 1C, p=0.04). We repeated this analysis in a subsequent cohort of 51 patients (clinical characteristics summarized in Table 3).

**Table 3**

| Clinical characteristics of 51 allo BMT patients transplanted at MSKCC with stool samples collected day +12 after BMT included in a validation cohort. | |
|---|---|
| Dates of transplant | August 2011 to August 2013 |
| Age (years) | 26 to 75, median 50 |
| Gender | Female 32%, male 69% |
| Primary malignancy | AML 37%, NHL 35%, ALL 12%, CLL 7.8%, Hodgkin disease 3.9%, MDS 3.9%, |
| Disease risk | High 35%, intermediate 27%, low 37% |
| Graft source | Bone marrow 3.9%, peripheral blood 57%, cord blood 39% |
| Donor relationship/HLA | Sibling identical (24%), unrelated identical (29%), unrelated non-identical (47%) |
| Conditioning intensity | standard intensity myeloablative 14%, reduced intensity myeloablative 57%, nonmyeloablative 29% |
| Stool sample collection day | +8 to +16, median +12 |

Despite differences in sequence methodology including analysis of V4-V5 of the 16S rRNA gene and using the MiSeq platform, this independent cohort demonstrated similar results, with a median *Blautia* abundance that was again 0.05% and confirmation of an association between *Blautia* abundance with less GVHD lethality (Figure 1C, p=0.01). Evaluating the combined cohorts, we found that *Blautia* abundance was strongly predictive of improved overall survival following allo BMT. This was largely driven by reduced GVHD-related mortality and to a lesser degree reduced relapse-related mortality (p=0.03), with no difference in non-GVHD treatment-related mortality (Figure 2). Similar results were obtained when cohorts were analyzed separately (data not shown). Adjusting for the two most readily modifiable risk factors for acute GVHD, graft source and conditioning intensity, we found that *Blautia* abundance maintained an association with reduced GVHD-related mortality (HR 0.13 [0.04-0.46], p=0.001). Regarding relapse-related mortality, an adjusted model factoring in disease risk and graft source demonstrated a reduction in the association with *Blautia* abundance (p=0.055).

We also evaluated the association of GVHD-related mortality with other bacteria. Because increased Enterococcus may be associated with GVHD³⁰, we evaluated if Enterococcus, or potentially beneficial bacteria (Lactobacillus and Bacteroides) were associated with GVHD-related mortality in our patient population. We also evaluated Veil-lonella, which was predicted by LEfSe analysis of the first patient flora cohort to be associated with increased GVHD-related mortality (p=0.047). Our results indicate that none of these bacterial taxa were predictive of GVHD-related mortality in the combined cohorts **(****Figure 5****).**

We also asked if bacterial subtypes related to *Blautia* could be predictive of reduced lethal GVHD. Bacteria from the genus *Blautia* are classified as follows: family-Lachnospiraceae, order - Clostridiales, class - Clostridia, and phylum - Firmicutes²⁸. Analyzing patients by abundance of bacteria from Lachnospiraceae, Clostridiales, and Clostridia all demonstrated associations with a reduced incidence of lethal GVHD, suggesting that members of *Blautia,* and potentially its relatives, contribute a protective effect against lethal GVHD (data not shown). At the species level, three *Blautia* taxa were associated with reduced GVHD-related mortality, similar to results at the genus level.

Having identified *Blautia* as a promising biomarker of GVHD-related mortality, we asked if it also correlated with reduced clinical acute GVHD. Our results indicate that *Blautia* abundance could be associated with a reduced incidence of acute GVHD grades 2-4 though this did not reach statistical significance (p=0.1); there was no association with acute GVHD grades 3-4 **(****Figure 3A****).** *Blautia* abundance did, however, predict for reduced development of acute GVHD requiring treatment with systemic corticosteroids (p=0.01), suggesting that loss of *Blautia* is associated with acute GVHD that will not respond to topical corticosteroids alone. Regarding classical acute GVHD target organs, increased *Blautia* abundance was not linked to skin GVHD or upper gut GVHD (Figure 3B). It trended towards being associated with reduced lower gut GVHD (p=0.1) and was associated with reduced liver GVHD (p=0.02) though the number of events was small.

We further examined the associations between *Blautia* abundance and GVHD outcomes while adjusting for clinical risk factors. After adjusting for the two most readily modifiable risk factors for acute GVHD, graft source and conditioning intensity, we found that *Blautia* abundance maintained an association with reduced GVHD leading to treatment with systemic steroids (HR 0.39 [0.19-0.78], p=0.009) and mortality (HR 0.13 [0.04-0.46], p=0.001). The limited number of events in our patient population precluded adjusting for additional factors. Corroborating this analysis, we found that in patients grouped by conditioning intensity, *Blautia* remained predictive for protection against lethal GVHD in patients with nonmyeloablative conditioning **(****Figure 7A****,** p=0.02) and trended towards being associated with protection in patients receiving myeloablative and reduced intensity conditioning, (p=0.1 and p=0.1). In patients grouped by graft source, those that received peripheral blood stem cell grafts showed a strong association between *Blautia* abundance and reduced lethal GVHD **(****Figure 7B****,** p=0.002), while those receiving cord blood stem cell grafts trended towards showing this association (p=0.2). Together, these results suggest that studies of larger cohorts of patients could demonstrate an association between *Blautia* abundance and reduced GVHD lethality across different conditioning intensities and graft sources.

### Blautia abundance is independent of known clinical acute GVHD risk factors

To determine if *Blautia* abundance provides additional prognostic information, we investigated potential associations between *Blautia* abundance and known risk factors for acute GVHD³¹⁻³³. We found that conditioning intensity, patient age, performance status, donor/patient gender, CMV status and disease risk were not associated with *Blautia* abundance (Table 4). While limited by small number, patients of an Asian or Hispanic background appeared to have lower abundance of *Blautia.* Finally, evaluating *Blautia* abundance and graft source also showed no association. In summary, our analysis indicates that *Blautia* abundance does not appear to be associated with known risk factors for acute GVHD.

**Table 4**

| Quantification of the association of known risk factors for acute GVHD and *Blautia* abundance | | | | | | |
|---|---|---|---|---|---|---|
| | | | | *Blautia* | | |
| | | N | | Mean (SD) | Median (range) | P-valu e |
| Graft source | PBSC | 6 | | 0.047 (0.094) | 0.001 (0-0.426) | 0.68 0* |
| | | 4 | | | | |
| | Cord blood | 4 | | 0.037 (0.088) | 0.001 (0-0.41) | |
| | | 7 | | | | |
| | BM | 4 | | 0.022 (0.043) | 0 (0-0.086) | |
| Donor relationship/HLA | PBSC sibling identical | 3 | | 0.068 (0.116) | 0.003 (0-0.426) | 0.68 6 |
| | | 0 | | | | |
| | PBSC unrelated identical | 2 | | 0.034 (0.069) | 0.001 (0-0.279) | |
| | | 9 | | | | |
| | Cord blood (unrelated non-identical) | 4 | | 0.037 (0.088) | 0.001 (0-0.41) | |
| | | 7 | | | | |
| Race | Asian/Hispanic | 1 | | 0.001 (0.003) | 0 (0-0.011) | 0.00 6 |
| | | 3 | | | | |
| | White/Black | 1 | | 0.047 (0.094) | 0.001 (0-0.426) | |
| | | 0 | | | | |
| | | 2 | | | | |
| Intensity | Myeloablative | 2 | | 0.01 (0.024) | 0(0-0.103) | 0.23 5 |
| | | 2 | | | | |
| | Reduced-intensity | 5 | | 0.03 (0.065) | 0.001 (0-0.365) | |
| | | 7 | | | | |
| | Nonmyeloablative | 3 | | 0.08 (0.129) | 0.001 (0-0.426) | |
| | | 6 | | | | |
| Age | 40 or younger | 2 | | 0.025 (0.053) | 0 (0-0.177) | 0.22 8 |
| | | 7 | | | | |
| | Over 40 | 8 | | 0.047 (0.098) | 0.001 (0-0.426) | |
| | | 8 | | | | |
| PS | 90 and above | 5 | | 0.05 (0.102) | 0.001 (0-0.426) | 0.41 2** |
| | | 8 | | | | |
| | Less than 90 | 5 | | 0.035 | 0 (0-0.365) | |
| | | 0 | | (0.076) | | |
| | Missing | 7 | | 0.028 (0.075) | 0(0-0.198) | |
| Sex (all transplants) | Female Donor/Male Pt | 3 | | 0.05 (0.096) | 0.001 (0-0.41) | 0.20 9 |
| | | 6 | | | | |
| | Other | 7 | | 0.038 (0.087) | 0(0-0.426) | |
| | | 9 | | | | |
| CMV | No positive | 4 | | 0.047 (0.084) | 0.001 (0-0.365) | 0.34 2** |
| | | 6 | | | | |
| | Any positive | 6 | | 0.041 (0.095) | 0(0-0.426) | |
| | | 6 | | | | |
| | Uncertain | 3 | | 0 | 0 (0-0) | |
| Disease risk | Low | 3 | | 0.049 (0.094) | 0.001 (0-0.426) | 0.32 5 |
| | | 1 | | | | |
| | Intermediate | 3 | | 0.045 (0.09) | 0.001 (0-0.365) | |
| | | 7 | | | | |
| | High | 4 | | 0.035 (0.088) | 0 (0-0.41) | |
| | | 7 | | | | |

| | | | | | | |
|---|---|---|---|---|---|---|
| PBSC = peripheral blood stem cell. BM = bone marrow. PS = performance status.*P-value compares *Blautia* levels across cord blood and PBSC groups. **Uncertain or missing covariate values not included in p-value calculation. | | | | | | |

### Identifying potential clinical determinants of Blautia abundance during allo BMT hospitalization

To better understand the heterogeneity in *Blautia* abundance in our patient population, we attempted to identify determinants of *Blautia* abundance. An analysis of all stool samples from both flora cohorts showed that a large majority of patients had relatively large amounts of *Blautia* upon admission for transplant hospitalization, with a median abundance of >0.1 (10%) **(****Figure 4A****).** In many patients, however, *Blautia* levels rapidly dropped during the course of hospitalization. As would be expected, we found that patients who were not exposed to antibiotics with anaerobic coverage were more likely to have increased levels of *Blautia* **(****Figure 4B****).**

Due to nausea and mucositis following conditioning, allo BMT patients commonly experience a prolonged period of significantly reduced oral intake and are treated with supplementary TPN. We used duration of TPN supplementation as an indicator of oral nutrition and examined for associations with *Blautia* abundance. Interestingly, patients with TPN use of less than 10 days duration (indicating delayed, interrupted or discontinued TPN therapy since initiation of TPN is considered on day 2 and stool samples were on average collected on day 12), had increased levels of *Blautia* **(****Figure 4B****).** TPN duration was associated with loss of *Blautia* even in patients that avoided treatment with anaerobe-active antibiotics **(****Figure 4C****).** Together these results indicate that anaerobic antibiotic therapy and poor oral nutritional intake both appear to mediate suppression of *Blautia* in the intestinal tract.

In this study we began with finding that in allo BMT recipients, the bacterial genus from stool samples most associated with reduced GVHD-related mortality was *Blautia,* in two independent cohorts. Patients with more *Blautia* also showed a reduced incidence of acute GVHD requiring treatment with systemic corticosteroids and improved overall survival. To demonstrate these associations, we ranked patients by their *Blautia* abundance and stratified by the median value, which happened to be 0.05% in both cohorts.

Surprisingly, despite the association with GVHD-related mortality, *Blautia* abundance did not distinguish the incidence of acute GVHD grades 3-4, which is known to identify patients less likely to respond to steroids, leading to poor survival³⁴. However, it is known that there is a subpopulation of patients initially presenting with grade 2 acute GVHD who nevertheless fare poorly that may be identified by novel GVHD grading systems³⁴ or by novel biomarkers³⁵. Further investigation in additional patient cohorts may determine if *Blautia* abundance can similarly add to the prognostic utility of clinical acute GVHD grading.

The abundance of bacteria from the class Clostridia, which includes *Blautia,* has also been shown to be predictive for reduced GVHD-related mortality in our patients. Interestingly, several of the 17 clostridial isolates are very close relatives of members of the genus *Blautia,* including one strain with a 16S sequence that most closely matches that of the 16S sequence (GenBank X94966) of species *Blautia producta* (ATCC^{R} 27340-DSM 2950, American Type Culture Collection, Manassas, VA), which was predictive for reduced GVHD lethality in our patient cohort. A beneficial anti-inflammatory association of *Blautia* has also been observed in other clinical settings, including colorectal cancer³⁶, inflammatory pouchitis following ileal pouch-anal anastomosis³⁶, and liver cirrhosis³⁷.

### Treatment with antibiotics with increased activity against anaerobes is associated with increased GVHD-related mortality and reduction of Clostridiales in allo-HSCT patients who develop neutropenic fever

In a second study, We began by asking if treatment with antibiotics that target anaerobic bacteria is associated with clinical differences in GVHD-related mortality. Allo-HSCT patients at our center receive a prophylactic regimen of antibiotics,
including a short course of trimethoprim-sulfamethoxazole to prevent *Pneumocystis jiroveci* pneumonia, as well as intravenous vancomycin and ciprofloxacin throughout the period of neutropenia. Notably, we have found that this regimen usually results in only mild perturbations to the composition of the intestinal microbiota (14). Later in the course of allo-HSCT, patients who develop neutropenic fever are treated with empiric antibiotics, the selection of which can vary due to a history of medication allergies or patient-specific considerations. Some patients who develop persistent fevers, abdominal symptoms, or have microbiological evidence of infection with a resistant bacterium may receive second-line antibiotics that are often more active against anaerobes. Finally, allo- HSCT patients are also commonly diagnosed with and treated for *Clostridium difficile* colitis during the allo-HSCT hospitalization, which rapidly leads to loss of anaerobic intestinal commensals (17, 18).

We retrospectively identified a cohort of 538 adult patients allo-HSCT patients consecutively transplanted at our center from 1994 to 2013 that met our inclusion criteria of being at standard risk for GVHD (i.e. no ex vivo T-cell depletion) and receiving treatment for neutropenic fever. Patients who received second-line antibiotics or received antibiotics that treat Clostridium difficile colitis (metronidazole either intravenously or orally, or vancomycin orally) were excluded. The remaining 283 patients were classified as receiving antibiotics that were more active against anaerobes (predominately piperacillin-tazobactam (pip/tazo) and imipenem-cilastatin (imipenem)), or receiving treatment with antibiotics less active against anaerobes (predominately cefepime and aztreonam) (19); clinical characteristics are provided in Table 2. We found that 225 patients who received antibiotics with anaerobic activity had a significantly increased incidence of GVHD-related mortality in the first year following allo-HSCT (Fig. 1A, p=0.04). Univariate analyses for previously identified GVHD risk factors demonstrated no significant association with GVHD-related mortality in this data set (Table 3), suggesting that the type of antibiotic exposure may be a novel predictor of GVHD-related mortality. We also performed a multivariate analysis evaluating the association of type of antibiotic exposure with GVHD related mortality while adjusting for GVHD risk factors associated with GVHD related mortality in our patient group using a significance criteria of p<0.1. We found that type of antibiotic exposure remained significant after adjusting for donor/HLA match (p=0.047) (Table 3). These results support the possibility that selecting antibiotics that preserve anaerobic commensals may reduce the risk of GVHD. An alternative hypothesis would be that patients with a history of allergies to penicillins (and are thus more likely to receive cephalosporins or aztreonam instead of penicillins and carbapenems) may be protected against GVHD, though this seems to have less biological plausibility.

HSCT patients were on intestinal bacterial composition. In 2009, our center began to prospectively collect weekly stool samples from patients undergoing allo-HSCT. From this specimen bank, we identified paired stool samples collected from patients prior to as well as following initiation of specific antibiotics during the course of allo-HSCT. Representative cases of patients treated for neutropenic fever, as well as of patients who did not require therapeutic antibiotics (but did receive prophylactic antibiotics), are shown in Fig.15, B to G. Using 16S rRNA gene deep sequencing, we evaluated the effects of these antibiotics on microbial composition. We focused on changes in abundance of *Clostridiales,* a predominant order of anaerobic gram-positive commensal bacteria that includes many species associated with intestinal health (8, 13, 20).

We found that patients often demonstrated loss of *Clostridiales* and this temporally coincided with beginning treatment with imipenem, pip/tazo, or metronidazole, while treatment with aztreonam often led to relative preservation of *Clostridiales* abundances (Fig. 15, B to G). Quantifying the change in *Clostridiales* abundance before and after starting specific antibiotics, we found that patients treated with imipenem, pip/tazo and metronidazole all had significantly lower abundances of *Clostridiales* compared to those treated with aztreonam (Fig. 15H).

### Treatment with imipenem (compared to aztreonam) is associated with increased disruption of the intestinal microbiota and exacerbated GVHD in mice

To further explore causality and mechanisms of the effects of antibiotics with anaerobic activity on GVHD, we turned to experiments in mice. We first treated healthy C57BL/6 mice with antibiotics either with increased activity against anaerobic bacteria (pip/tazo, imipenem, and metronidazole) or with reduced activity (aztreonam and cefepime). Mice were treated by subcutaneous (SC) injections of each antibiotic twice a day for two days (500 mg/kg for pip/tazo and 100 mg/kg for others) and stool samples were collected, followed by 16S rRNA gene amplification and sequence analysis. We found that systemic treatment with imipenem or metronidazole significantly reduced the abundance of Clostridiales and increased that of Enterococcus, while treatment with aztreonam or cefepime spared Clostridiales (Fig. 16A). Interestingly, treatment with pip/tazo resulted in no amplifiable bacterial DNA after two days of treatment, indicating near-complete decontamination in mice (data not shown).

We next investigated the effects of antibiotic treatment in a clinically relevant MHC-matched minor antigen-mismatched allo-HSCT model (C57BL/6 into 129S1). We chose not to administer prophylactic antibiotics such as IV vancomycin or ciprofloxacin, which minimally perturb the intestinal microbiota composition, and focused on comparing the effects of aztreonam, which spared Clostridiales in both patients and mice, with imipenem, which depleted Clostridiales in both patients and mice, when given in the first weeks after allo-HSCT similar to the frequent clinical scenario of post-transplant fever/neutropenia. Lethally irradiated 129S1 recipients were transplanted with C57BL/6 T-cell depleted bone marrow (TCD-BM) cells and 1 × 106 C57BL/6 splenic T cells. Control recipients received TCD-BM only. Recipients were treated with either aztreonam or imipenem SC three times per week starting on day 10 after allo-HSCT. Remarkably, we observed significantly increased mortality in imipenem-treated recipients within 2 weeks of starting treatment (Fig. 16B). Control recipients without T-cell transfer (no GVHD control) showed 100% survival, indicating that antibiotic treatment itself did not have adverse effects on BM engraftment or survival after myeloablative irradiation. These results were reproducible in three consecutive and independent experiments. Histological examination of GVHD target organs on day 21 after allo-HSCT (11 days after starting antibiotic therapy) revealed that increased GVHD pathology was present in mice treated with imipenem. Interestingly, this was localized to the colon (Fig. 16C and Fig. 18) while other common GVHD target organs, including the skin, liver and small intestine, showed no significant differences in the degree of inflammation and damage. 16S rRNA gene sequencing of stool samples from mice with GVHD followed by principal component analysis indicated that aztreonam and imipenem therapy led to distinct patterns of microbiota composition (Fig. 16D). The taxa that best explained the differences between these groups, as assessed by linear discriminate analysis of effect size (LEfSe) (21) are depicted in Fig. 16, E and F. Transplanted mice treated with imipenem showed a loss of *Clostridiales,* corroborating our results in patients and untransplanted mice.

### Increase in Akkermansia municiphila

Interestingly, an expansion of *Akkermansia muciniphila* was observed consistently in six experiments in these animals (Fig. 16, G and H). We evaluated the effects of imipenem treatment on T-cell infiltration and STAT3 phosphorylation in the colon of mice with GVHD and found increased numbers of T cells infiltrating the colon, both by flow cytometry and by histology, with significantly higher levels of phosphorylated STAT3 seen in T cells in situ by fluorescent microscopy, supporting the notion that elevated levels of IL-23 participated in the recruitment and activation of donor T-cells, which likely contributed to aggravated GVHD specifically in the colon. In mice with GVHD, imipenem treatment led to an expansion of *Akkermansia muciniphila,* a common commensal bacteria found in the intestinal tract of humans, mice and other animals. Notably, this bacterium is unusual in its ability to utilize mucin as a source of carbon and nitrogen (33). Breakdown of the colonic mucus layer has been observed following mono-colonization of germ-free mice with *Akkermansia muciniphila,* suggesting that *Akkermansia muciniphila* can degrade mucin in vivo as well as in vitro (51). What the effects of *Akkermansia muciniphila* are on intestinal homeostasis, however, is less clear and likely setting-dependent. In a murine obesity model, treatment with *Akkermansia muciniphila* resulted in improvement of metabolic disorders and reduced systemic levels of endotoxins, suggesting that in this setting *Akkermansia muciniphila* improved intestinal barrier function (52). However, a gnotobiotic murine model of Salmonella typhimurium infection showed that presence of *Akkermansia muciniphila* led to aggravated intestinal inflammation that was attributed to colonic mucus disruption (53). Our examination of the colon in imipenem-treated animals demonstrated a nearly complete effacement of the mucus layer. We also detected the presence of bacteria in the colonic lamina propria beyond the disrupted mucus layer, which is in agreement with the mucus layer providing a critical first line of defense against invasion of the intestinal mucosa (54). Why *Akkermansia* expands in the colon of transplanted mice treated with imipenem is unclear; to our knowledge, *Akkermansia* isolates have not been noted to be resistant to imipenem or other related antibiotics. Competitive interactions between *Akkermansia* and *Clostridiales* have also to our knowledge not been described, though studies have seen similar expansions of *Akkermansia* following treatment of mice with other antibiotics that inhibit *Clostridiales,* such as clindamycin (55). These results suggest that selecting antibiotics with a more limited spectrum of activity (especially against anaerobes) can prevent microbiota injury and reduce GVHD.

*Clostridiales* have notably been identified as major producers of short-chain fatty acids (SCFA) (10, 22), which are bacterial fermentation products that play an important role in maintaining colonic homeostasis and health (23, 24). Surprisingly, despite large differences in the abundances of *Clostridiales,* we observed no significant changes in the levels of SCFA in the colon comparing samples from recipients treated with aztreonam or imipenem (data not shown).

In order to acquire greater resolution of the bacterial composition between aztreonam- and imipenem-treated subject samples, we performed metagenomics shotgun sequencing with stool collected on day 21 after allo-HSCT. Our findings revealed that, concordant with the 16S sequencing results, imipenem but not aztreonam treatment resulted in an increased abundance of *Akkermansia muciniphilia* (Fig. 16I). However, as the largest percentage of reads from the analysis were determined to be unclassified, it is possible that additional significant differences in bacterial species composition exist between the two antibiotic-treatment types. Metagenomic shotgun sequencing analysis also revealed differences in gene content between microbiota samples from mice treated with aztreonam and imipenem, depicted by principal component analysis of gene orthologs (Fig. 16J). LEfSe analysis of gene pathways indicated that the microbiota genes in mice treated with imipenem were enriched for processes including lipopolysaccharide synthesis, and relatively depleted in several processes including D-alanine metabolism (data not shown). Interestingly, lipopolysaccharide is well-known for inducing a pro-inflammatory cascade in many disease processes including GVHD (25), while reductions in D-alanine content of lipotechoic acid can enhance the anti-inflammatory properties of *Lactobacilli* (26, 27).

As mentioned above, we detected an increase in *Akkermansia muciniphila* in the flora of imipenem-treated mice using 16S rRNA deep sequencing (Fig. 16H). This bacterium has the ability to degrade mucus as a carbohydrate source (33, 34). Utilizing our metagenomic shotgun sequencing results, we asked if genes predicted to encode for secretory mucolytic enzymes were differentially present in mice treated with each antibiotic. The identification and characterization of bacterial mucolytic enzymes is still a young field, but a recent study examined the whole genomic sequence of *Akkermansia muciniphila* ATCC BAA-835, isolated from human feces (34). The authors identified two strong candidates for mucus degradation: Amuc_0953, a sulfatase, and Amuc_2164, a glycosyl hydrolase, which both contained predicted secretory signal peptide cleavage sites as well as predicted mucin-binding domains. We quantified the presence of sequences with homology to these two genes and found that both were markedly enriched in samples from imipenem-treated mice (Fig. 17G). We then sought to characterize the mucus layer of the colon in antibiotic-treated transplanted mice. Using Periodic acid-Schiff staining, we observed a marked reduction in the thickness of the mucus layer in recipients treated with imipenem on day 21 compared to aztreonam-treated recipients (Fig. 17, H and I). No differences in the numbers of mucus-producing goblet cells between aztreonam and imipenem-treated recipients were seen suggesting that mucus production was not impaired (Fig. 17J). Moreover, by utilizing a general bacterial 16S rRNA probe (EUB338) (35) coupled with Muc2 staining, we directly visualized the inner mucus layer in the colon of antibiotic-treated recipients and confirmed a dramatic thinning of the mucus layer of mice treated with imipenem. Strikingly, we also histologically observed dissemination of bacteria past the colonic epithelial barrier in imipenem-treated mice (Fig. 17K), while this was not seen in aztreonam treated mice. Taken together, these results indicate that imipenem treatment mayexacerbate GVHD through a combination of factors including compromised barrier function with thinning of the protective mucus layer and reduced numbers of colonic B cells, increased infiltration with granulocytes, elevated levels of IL-23, and increased numbers and activation of donor effector CD4+ T cells.

One question that arises is how the abundance of *Blautia* and other related bacteria as early as day 12 after allo BMT could biologically impact on acute GVHD and GVHD-related mortality, which can occur months, and in the case of mortality, years, after allo BMT. There are precedents however; serum cyclosporine concentrations in the first week following allo BMT predicted for onset of acute GVHD, even though onset of acute GVHD largely occurred after day 30³⁸. Similarly, serum levels of the biomarker ST2 predicted for steroid-refractory GVHD and levels as early as day 14 were associated with 6-month mortality without relapse³⁵. Together, these studies suggest that conditions early post-BMT may affect the initiation of GVHD and dictate the eventual severity of the course of GVHD, though this can take months to fully manifest, perhaps due to partial containment of inflammation by ongoing administration of immune suppressants in the forms of GVHD prophylaxis and therapy.

Interestingly, while our results suggest that *Blautia* is associated with reduced GVHD, increased abundance of *Blautia* was not associated with increased relapse-related mortality. This suggests that *Blautia* may be linked with primarily localized anti-inflammatory effects, a possibility supported by our finding of a lack of an association with skin GVHD. Together, these data suggest that targeting the microbiota may allow for reduced GVHD without simultaneously compromising graft-versus-tumor effects. Indeed, we found an association of *Blautia* abundance with reduced relapse-related mortality, although this association was lost after adjusting for disease risk and graft source. Examining more thoroughly the impact of the microbiota on relapse would require further study.

Characterizing the abundance of *Blautia* in our patients over the course of their transplant hospitalization, we found that most patients initially had relatively large amounts of *Blautia,* but in many patients *Blautia* species were then dramatically lost. We identified two potential risk factors associated with loss of *Blautia,* including receiving antibiotics with anaerobic coverage and requiring longer treatment duration of TPN. The finding of reduced *Blautia* with antibiotic administration is not surprising, but the association with prolonged TPN was unexpected. While conditioning intensity and duration of TPN necessity are known to be associated³⁹, we found no significant association between conditioning intensity and *Blautia* abundance (data not shown). This suggests that poor oral nutrition may be a more likely contributor to loss of *Blautia* than more intense conditioning. This explanation is corroborated by findings in mouse models that myeloablative conditioning is associated with only mild perturbations in flora composition, in comparison to larger perturbations characterized by loss of Clostridiales seen in both mice and humans with the onset of GVHD, a potent inducer of anorexia⁴⁰. A pattern of loss of members of Clostridiales, including *Roseburia, Faecalibacterium, Ruminococcus* and *Blautia* species, can similarly be observed in volunteers placed on high-protein and low-carbohydrate diets⁴¹ or on diets derived entirely from animal products⁴².

From the foregoing detailed description of the specific embodiments of the present invention, it should be readily apparent that a unique methodology for the utilization of Clostridiales bacteria for reducing the risk and/or treating GVHD following bone marrow or hematopoietic stem cell transplant has been described. Although particular embodiments have been disclosed herein in detail, this has been done by way of example for purposes of illustration and is not intended to be limiting with respect to the scope of the appended claims. The references to the methods of treatment by therapy or surgery or in vivo diagnosis methods in this description are to be interpreted as references to compounds, pharmaceutical compositions and medicaments of the present invention for use in those methods.

### References

### References

1. Pasquini MC WZ. Current use and outcome of hematopoietic stem cell transplantation. CIBMTR Summary Slides, 2013. Available at: http://www.cibmtr.org.
2. Jones JM, Wilson R, Bealmear PM. Mortality and gross pathology of secondary disease in germfree mouse radiation chimeras. Radiat Res. 1971;45(3):577-588.
3. van Bekkum DW, Roodenburg J, Heidt PJ, van der Waaij D. Mitigation of secondary disease of allogeneic mouse radiation chimeras by modification of the intestinal microflora. J Natl Cancer Inst. 1974;52(2):401-404.
4. Storb R, Prentice RL, Buckner CD, et al. Graft-versus-host disease and survival in patients with aplastic anemia treated by marrow grafts from HLA-identical siblings. Beneficial effect of a protective environment. N Engl J Med. 1983;308(6):302-307.
5. Vossen JM, Heidt PJ, van den Berg H, Gerritsen EJ, Hermans J, Dooren LJ. Prevention of infection and graft-versus-host disease by suppression of intestinal microflora in children treated with allogeneic bone marrow transplantation. EurJ Clin Microbiol Infect Dis. 1990;9(1):14-23.
6. Petersen FB, Buckner CD, Clift RA, et al. Infectious complications in patients undergoing marrow transplantation: a prospective randomized study of the additional effect of decontamination and laminar airflow isolation among patients receiving prophylactic systemic antibiotics. ScandJ Infect Dis. 1987;19(5):559-567.
7. Passweg JR, Rowlings PA, Atkinson KA, et al. Influence of protective isolation on outcome of allogeneic bone marrow transplantation for leukemia. Bone Marrow Transplant. 1998;21(12):1231-1238.
8. Russell JA, Chaudhry A, Booth K, et al. Early outcomes after allogeneic stem cell transplantation for leukemia and myelodysplasia without protective isolation: a 10-year experience. Biol Blood Marrow Transplant. 2000;6(2):109-114.
9. Martin PJ, McDonald GB, Sanders JE, et al. Increasingly frequent diagnosis of acute gastrointestinal graft versus-host disease after allogeneic hematopoietic cell transplantation Biol Blood Marrow Transplant. 2004;10(5):320-327.
10. Beelen DW, Elmaagacli A, Muller KD, Hirche H, Schaefer UW. Influence of intestinal bacterial decontamination using metronidazole and ciprofloxacin or ciprofloxacin alone on the development of acute graft-versus-host disease after marrow transplantation in patients with hematologic malignancies: final results and long-term follow-up of an open-label prospective randomized trial. Blood. 1999;93(10):3267-3275.
11.Taur Y, Xavier JB, Lipuma L, et al. Intestinal Domination and the Risk of Bacteremia in Patients Undergoing Allogeneic Hematopoietic Stem Cell Transplantation. Clin Infect Dis. 2012.
12.Atarashi K, Tanoue T, Oshima K, et al. Treg induction by a rationally selected mixture of Clostridia strains from the human microbiota. Nature. 2013;500(7461):232-236.
13. Taur Y, Jenq RR, Perales MA, et al. The effects of intestinal tract bacterial diversity on mortality following allogeneic hematopoietic stem cell transplantation. Blood 124, 1174-1182 (2014).
14. Jakubowski AA, Small TN, Kernan NA, et al. T cell-depleted unrelated donor stem cell transplantation provides favorable disease-free survival for adults with hematologic malignancies. Biol Blood Marrow Transplant. 2011;17(9):1335-1342.
15. Goldberg JD, Linker A, Kuk D, et al. T cell-depleted stem cell transplantation for adults with high-risk acute lymphoblastic leukemia: long-term survival for patients in first complete remission with a decreased risk of graft-versus-host disease. Biol Blood Marrow Transplant. 2013;19(2):208-213.
16. Rowlings PA, Przepiorka D, Klein JP, et al. IBMTR Severity Index for grading acute graft-versus-host disease: retrospective comparison with Glucksberg grade. BrJ Haematol. 1997;97(4):855-864.
17.Vigorito AC, Campregher PV, Storer BE, et al. Evaluation of NIH consensus criteria for classification of late acute and chronic GVHD. Blood. 2009;114(3):702-708.
18. Ponce DM, Gonzales A, Lubin M, et al. Graft-versus-host disease after double-unit cord blood transplantation has unique features and an association with engrafting unit-to-recipient HLA match. Biol Blood Marrow Transplant. 2013;19(6):904-911.
19. Copelan E, Casper JT, Carter SL, et al. A scheme for defining cause of death and its application in the Tcell depletion trial. Biol Blood Marrow Transplant. 2007;13(12):1469-1476.
20. Transplantation ASfBaM. ASBMT RFI 2014 - Disease Classifications Corresponding to CIBMTR Classifications. 2014.
21. Bacigalupo A, Ballen K, Rizzo D, et al. Defining the intensity of conditioning regimens: working definitions. Biol Blood Marrow Transplant. 2009;15(12):1628-1633.
22.Solomkin JS, Mazuski JE, Bradley JS, et al. Diagnosis and management of complicated intra-abdominal infection in adults and children: guidelines by the Surgical Infection Society and the Infectious Diseases Society of America. Clin Infect Dis. 2010;50(2):133-164.
23. iaffe D, iakubowski A, Sepkowitz K, et al. Prevention of peritransplantation viridans streptococcal bacteremia with early vancomycin administration: a single-center observational cohort study. Clin Infect Dis. 2004;39(11):1625-1632.
24. Turnbaugh Pi, Hamady M, Yatsunenko T, et al. A core gut microbiome in obese and lean twins. Nature. 2009;457(7228):480-484.
25. Schloss PD, Westcott SL, Ryabin T, et al. Introducing mothur: open-source, platform-independent, community-supported software for describing and comparing microbial communities. Appl Environ Microbiol. 2009;75(23):7537-7541.
26. Caporaso JG, Kuczynski J, Stombaugh J, et al. QIIME allows analysis of high-throughput community sequencing data. Nat Methods. 2010;7(5):335-336.
27. Schloss PD, Gevers D, Westcott SL. Reducing the effects of PCR amplification and sequencing artifacts on 16S rRNA-based studies. PLoS One. 2011;6(12):e27310.
28. Wang Q, Garrity GM, Tiedje JM, Cole JR. Naive Bayesian classifier for rapid assignment of rRNA sequences into the new bacterial taxonomy. Appl Environ Microbiol. 2007;73(16):5261-5267.
29. DeSantis TZ, Hugenholtz P, Larsen N, et al. Greengenes, a chimera-checked 16S rRNA gene database and workbench compatible with ARB. Appl Environ Microbiol. 2006;72(7):5069-5072.
30. Magurran AE. Measuring biological diversity. Malden, MA: Blackwell Publishing; 2004.
31.Shannon CE. The mathematical theory of communication. 1963. MD Comput. 1997;14(4):306-317.
32.Segata N, Izard J, Waldron L, et al. Metagenomic biomarker discovery and explanation. Genome Biol. 2011;12(6):R60.
33. Clifford Ri, Milillo M, Prestwood i, et al. Detection of bacterial 16S rRNA and identification of four clinically important bacteria by real-time PCR. PLoS One. 2012;7(11):e48558.
34.Ahmad T, Fiuzat M, Neely B, et al. Biomarkers of myocardial stress and fibrosis as predictors of mode of death in patients with chronic heart failure. JACC Heart Fail. 2014;2(3):260-268.
35. Liu C, Finegold SM, Song Y, Lawson PA. Reclassification of Clostridium coccoides, Ruminococcus hansenii, Ruminococcus hydrogenotrophicus, Ruminococcus luti, Ruminococcus productus and Ruminococcus schinkii as Blautia coccoides gen. nov., comb. nov., Blautia hansenii comb. nov., Blautia hydrogenotrophica comb. nov., Blautia luti comb. nov., Blautia producta comb. nov., Blautia schinkii comb. nov. and description of Blautia wexlerae sp. nov., isolated from human faeces. IntJ Syst Evol Microbiol. 2008;58(Pt 8):1896-1902.
36. Park SK, Kim MS, Bae JW. Blautia faecis sp. nov., isolated from human faeces. Int J Syst Evol Microbiol. 2013;63(Pt 2):599-603.
37. Holler E, Butzhammer P, Schmid K, et al. Metagenomic Analysis of the Stool Microbiome in Patients Receiving Allogeneic Stem Cell Transplantation: Loss of Diversity Is Associated with Use of Systemic Antibiotics and More Pronounced in Gastrointestinal Graft-versus-Host Disease. Biol Blood Marrow Transplant. 2014;20(5):640-645.
38.Atarashi K, Tanoue T, Shima T, et al. Induction of colonic regulatory T cells by indigenous Clostridium species. Science. 2011;331(6015):337-341.
39. Collins MD, Lawson PA, Willems A, et al. The phylogeny of the genus Clostridium: proposal of five new genera and eleven new species combinations. IntJ Syst Bacteriol. 1994;44(4):812-826.
40. Weisdorf D, Hakke R, Blazar B, et al. Risk factors for acute graft-versus-host disease in histocompatible donor bone marrow transplantation. Transplantation. 1991;51(6):1197-1203.
41. iagasia M, Arora M, Flowers ME, et al. Risk factors for acute GVHD and survival after hematopoietic cell transplantation. Blood. 2012;119(1):296-307.
42. Hahn T, McCarthy PL, Jr., Zhang MJ, et al. Risk factors for acute graft-versus-host disease after human leukocyte antigen-identical sibling transplants for adults with leukemia. J Clin Oncol. 2008;26(35):5728-5734.
43. MacMillan ML, DeFor TE, Weisdorf DJ. What predicts high risk acute graft-versus-host disease (GVHD) at onset?: identification of those at highest risk by a novel acute GVHD risk score. BrJ Haematol. 2012;157(6):732 741.
44.Vander Lugt MT, Braun TM, Hanash S, et al. ST2 as a marker for risk of therapy-resistant graft-versus host disease and death. N Engl J Med. 2013;369(6):529-539.
45.Chen W, Liu F, Ling Z, Tong X, Xiang C. Human intestinal lumen and mucosa-associated microbiota in patients with colorectal cancer. PLoS One. 2012;7(6):e39743.
46. Bajaj JS, Hylemon PB, Ridlon JM, et al. Colonic mucosal microbiome differs from stool microbiome in cirrhosis and hepatic encephalopathy and is linked to cognition and inflammation. Am J Physiol Gastrointest Liver Physiol. 2012;303(6):G675-685.
47. Malard F, Szydlo RM, Brissot E, et al. Impact of cyclosporine-A concentration on the incidence of severe acute graft-versus-host disease after allogeneic stem cell transplantation. Biol Blood Marrow Transplant. 2010;16(1):28-34.
48. Barrell C, Dietzen D, Jin Z, Pinchefsky S, Petrillo K, Satwani P. Reduced-intensity conditioning allogeneic stem cell transplantation in pediatric patients and subsequent supportive care. Oncol Nurs Forum. 2012;39(6):E451-458.
49. Jenq RR, Ubeda C, Taur Y, et al. Regulation of intestinal inflammation by microbiota following allogeneic bone marrow transplantation. J Exp Med. 2012;209(5):903-911.
50. Russell WR, Gratz SW, Duncan SH, et al. High-protein, reduced-carbohydrate weight-loss diets promote metabolite profiles likely to be detrimental to colonic health. Am J Clin Nutr. 2011;93(5):1062-1072.
51. David LA, Maurice CF, Carmody RN, et al. Diet rapidly and reproducibly alters the human gut microbiome. Nature. 2014;505(7484):559-563.
52. Gallo RL, Hooper LV. Epithelial antimicrobial defence of the skin and intestine. Nat Rev Immunol. 2012;12(7):503-516.
53. Seguy D, Berthon C, Micol JB, et al. Enteral feeding and early outcomes of patients undergoing allogeneic stem cell transplantation following myeloablative conditioning. Transplantation. 2006;82(6):835-839
54. Jaffe D, Jakubowski A, Sepkowitz K, et al. Prevention of peritransplantation viridans streptococcal bacteremia with early vancomycin administration: a single-center observational cohort study. Clin Infect Dis. 2004;39(11):1625-1632.
55. Jagasia M, Arora M, Flowers ME, et al. Risk factors for acute GVHD and survival after hematopoietic cell transplantation. Blood. 2012;119(1):296-307.
56. J. D. Meyers, Infection in bone marrow transplant recipients. The American journal of medicine 81, 27-38 (1986).
57. J. R. Wingard, Opportunistic infections after blood and marrow transplantation. Transplant infectious disease : an official journal of the Transplantation Society 1, 3-20 (1999).
58. M. T. LaRocco, S. J. Burgert, Infection in the bone marrow transplant recipient and role of the microbiology laboratory in clinical transplantation. Clinical microbiology reviews 10, 277-297 (1997).
59. J. W. Hiemenz, Management of infections complicating allogeneic hematopoietic stem cell transplantation. Seminars in hematology 46, 289-312 (2009).
60. A. G. Freifeld, E. J. Bow, K. A. Sepkowitz, M. J. Boeckh, J. I. Ito, C. A. Mullen, Raad, II, K. V. Rolston, J. A. Young, J. R. Wingard, A. Infectious Diseases Society of, Clinical practice guideline for the use of antimicrobial agents in neutropenic patients with cancer: 2010 update by the infectious diseases society of america. Clinical infectious diseases: an official publication of the Infectious Diseases Society ofAmerica 52, e56-93 (2011).
61. C. L. Maynard, C. O. Elson, R. D. Hatton, C. T. Weaver, Reciprocal interactions of the intestinal microbiota and immune system. Nature 489, 231-241 (2012).
62. C. G. Buffie, E. G. Pamer, Microbiota-mediated colonization resistance against intestinal pathogens. Nature reviews. Immunology 13, 790-801 (2013).
63. K. Atarashi, T. Tanoue, T. Shima, A. Imaoka, T. Kuwahara, Y. Momose, G. Cheng, S. Yamasaki, T. Saito, Y. Ohba, T. Taniguchi, K. Takeda, S. Hori, Ivanov, II, Y. Umesaki, K. Itoh, K. Honda, Induction of colonic regulatory T cells by indigenous Clostridium species. Science 331, 337-341 (2011).
64. S. Narushima, Y. Sugiura, K. Oshima, K. Atarashi, M. Hattori, M. Suematsu, K. Honda, Characterization of the 17 strains of regulatory T cell-inducing human-derived Clostridia. Gut microbes 5, 333-339 (2014).
65. K. Atarashi, T. Tanoue, K. Oshima, W. Suda, Y. Nagano, H. Nishikawa, S. Fukuda, T. Saito, S. Narushima, K. Hase, S. Kim, J. V. Fritz, P. Wilmes, S. Ueha, K. Matsushima, H. Ohno, B. Olle, S. Sakaguchi, T. Taniguchi, H. Morita, M. Hattori, K. Honda, Treg induction by a rationally selected mixture of Clostridia strains from the human microbiota. Nature 500, 232-236 (2013).
66.Y. Eriguchi, S. Takashima, H. Oka, S. Shimoji, K. Nakamura, H. Uryu, S. Shimoda, H. Iwasaki, N. Shimono, T. Ayabe, K. Akashi, T. Teshima, Graft versus-host disease disrupts intestinal microbial ecology by inhibiting Paneth cell production of alpha-defensins. Blood 120, 223-231 (2012).
67.Y. Shono, M. D. Docampo, J. U. Peled, S. M. Perobelli, R. R. Jenq, Intestinal microbiota-related effects on graft-versus-host disease. International journal of hematology 101, 428-437 (2015).
68. R. Jenq, Y. Taur, S. Devlin, D. Ponce, J. Goldberg, K. F. Ahr, E. R. Littmann, L. Ling, A. C. Gobourne, L. C. Miller, M. D. Docampo, J. U. Peled, N. Arpaia, J. Cross, T. K. Peets, M. A. Lumish, Y. Shono, J. A. Dudakov, H. Poeck, A. M. Hanash, J. N. Barker, M. A. Perales, S. A. Giralt, E. G. Pamer, M. R. van den Brink, Intestinal Blautia Is Associated with Reduced Death from Graft-versus-Host Disease. Biology of blood and marrow transplantation : journal of the American Society for Blood and Marrow Transplantation, (2015).
69. M. A. Kinnebrew, Y. J. Lee, R. R. Jenq, L. Lipuma, E. R. Littmann, A. Gobourne, D. No, M. van den Brink, E. G. Pamer, Y. Taur, Early Clostridium difficile infection during allogeneic hematopoietic stem cell transplantation. PloS one 9, e90158 (2014).
70.Y. Furusawa, Y. Obata, S. Fukuda, T. A. Endo, G. Nakato, D. Takahashi, Y. Nakanishi, C. Uetake, K. Kato, T. Kato, M. Takahashi, N. N. Fukuda, S. Murakami, E. Miyauchi, S. Hino, K. Atarashi, S. Onawa, Y. Fujimura, T. Lockett, J. M. Clarke, D. L. Topping, M. Tomita, S. Hori, O. Ohara, T. Morita, H. Koseki, J. Kikuchi, K. Honda, K. Hase, H. Ohno, Commensal microbe-derived butyrate induces the differentiation of colonic regulatory T cells. Nature 504, 446-450 (2013).
71. P. M. Smith, M. R. Howitt, N. Panikov, M. Michaud, C. A. Gallini, Y. M. Bohlooly, J. N. Glickman, W. S. Garrett, The microbial metabolites, short-chain fatty acids, regulate colonic Treg cell homeostasis. Science 341, 569-573 (2013).
72. N. Arpaia, C. Campbell, X. Fan, S. Dikiy, J. van der Veeken, P. deRoos, H. Liu, J. R. Cross, K. Pfeffer, P. J. Coffer, A. Y. Rudensky, Metabolites produced by commensal bacteria promote peripheral regulatory T-cell generation. Nature 504, 451-455 (2013).
73. K. R. Cooke, A. Gerbitz, J. M. Crawford, T. Teshima, G. R. Hill, A. Tesolin, D. P. Rossignol, J. L. Ferrara, LPS antagonism reduces graft-versus-host disease and preserves graft-versus-leukemia activity after experimental bone marrow transplantation. The Journal of clinical investigation 107, 1581-1589 (2001).
74. S. C. Duncker, L. Wang, P. Hols, J. Bienenstock, The D-alanine content of lipoteichoic acid is crucial for Lactobacillus plantarum-mediated protection from visceral pain perception in a rat colorectal distension model. Neurogastroenterology and motility : the official journal of the European Gastrointestinal Motility Society 20, 843-850 (2008).
75. C. Grangette, S. Nutten, E. Palumbo, S. Morath, C. Hermann, J. Dewulf, B. Pot, T. Hartung, P. Hols, A. Mercenier, Enhanced antiinflammatory capacity of a Lactobacillus plantarum mutant synthesizing modified teichoic acids. Proceedings of the National Academy of Sciences of the United States of America 102, 10321-10326 (2005).
76. R. Das, X. Chen, R. Komorowski, M. J. Hessner, W. R. Drobyski, Interleukin-23 secretion by donor antigen-presenting cells is critical for organ-specific pathology in graft-versus-host disease. Blood 113, 2352-2362 (2009).
77. R. Das, R. Komorowski, M. J. Hessner, H. Subramanian, C. S. Huettner, D. Cua, W. R. Drobyski, Blockade of interleukin-23 signaling results in targeted protection of the colon and allows for separation of graft-versus-host and graft-versus-leukemia responses. Blood 115, 5249-5258 (2010).
78. L. Schwab, L. Goroncy, S. Palaniyandi, S. Gautam, A. Triantafyllopoulou, A. Mocsai, W. Reichardt, F. J. Karlsson, S. V. Radhakrishnan, K. Hanke, A. Schmitt Graeff, M. Freudenberg, F. D. von Loewenich, P. Wolf, F. Leonhardt, N. Baxan, D. Pfeifer, O. Schmah, A. Schonle, S. F. Martin, R. Mertelsmann, J. Duyster, J. Finke, M. Prinz, P. Henneke, H. Hacker, G. C. Hildebrandt, G. Hacker, R. Zeiser, Neutrophil granulocytes recruited upon translocation of intestinal bacteria enhance graft-versus-host disease via tissue damage. Nature medicine 20, 648-654 (2014).
79.Y. Shono, S. Shiratori, M. Kosugi-Kanaya, S. Ueha, J. Sugita, A. Shigematsu, T. Kondo, D. Hashimoto, K. Fujimoto, T. Endo, M. Nishio, S. Hashino, Y. Matsuno, K. Matsushima, J. Tanaka, M. Imamura, T. Teshima, Bone marrow graft versus-host disease: evaluation of its clinical impact on disrupted hematopoiesis after allogeneic hematopoietic stem cell transplantation. Biology of blood and marrow transplantation : journal of the American Society for Blood and Marrow Transplantation 20, 495-500 (2014).
80. C. Lindner, I. Thomsen, B. Wahl, M. Ugur, M. K. Sethi, M. Friedrichsen, A. Smoczek, S. Ott, U. Baumann, S. Suerbaum, S. Schreiber, A. Bleich, V. Gaboriau-Routhiau, N. Cerf-Bensussan, H. Hazanov, R. Mehr, P. Boysen, P. Rosenstiel, O. Pabst, Diversification of memory B cells drives the continuous adaptation of secretory antibodies to gut microbiota. Nature immunology, (2015).
81. M. Derrien, M. W. van Passel, J. H. van de Bovenkamp, R. G. Schipper, W. M. de Vos, J. Dekker, Mucin-bacterial interactions in the human oral cavity and digestive tract. Gut microbes 1, 254-268 (2010).
82. M. W. van Passel, R. Kant, E. G. Zoetendal, C. M. Plugge, M. Derrien, S. A. Malfatti, P. S. Chain, T. Woyke, A. Palva, W. M. de Vos, H. Smidt, The genome of Akkermansia muciniphila, a dedicated intestinal mucin degrader, and its use in exploring intestinal metagenomes. PloS one 6, e16876 (2011).
83. S. Vaishnava, M. Yamamoto, K. M. Severson, K. A. Ruhn, X. Yu, O. Koren, R. Ley, E. K. Wakeland, L. V. Hooper, The antibacterial lectin Reglllgamma promotes the spatial segregation of microbiota and host in the intestine. Science 334, 255-258 (2011).
84. J. M. Vossen, H. F. Guiot, A. C. Lankester, A. C. Vossen, R. G. Bredius, R. Wolterbeek, H. D. Bakker, P. J. Heidt, Complete suppression of the gut microbiome prevents acute graft-versus-host disease following allogeneic bone marrow transplantation. PloS one 9, e105706 (2014).
85. D. Weber, P. J. Oefner, A. Hiergeist, J. Koestler, A. Gessner, M. Weber, J. Hahn, D. Wolff, F. Stammler, R. Spang, W. Herr, K. Dettmer, E. Holler, Low urinary indoxyl sulfate levels early after ASCT reflect a disrupted microbiome and are associated with poor outcome. Blood, (2015).
86. M. Kamboj, D. Chung, S. K. Seo, E. G. Pamer, K. A. Sepkowitz, A. A. Jakubowski, G. Papanicolaou, The changing epidemiology of vancomycin-resistant Enterococcus (VRE) bacteremia in allogeneic hematopoietic stem cell transplant (HSCT) recipients. Biology of blood and marrow transplantation: journal of the American Society for Blood and Marrow Transplantation 16, 1576-1581 (2010).
87. P. P. Ahern, A. Izcue, K. J. Maloy, F. Powrie, The interleukin-23 axis in intestinal inflammation. Immunological reviews 226, 147-159 (2008).
88. S. Hue, P. Ahern, S. Buonocore, M. C. Kullberg, D. J. Cua, B. S. McKenzie, F. Powrie, K. J. Maloy, Interleukin-23 drives innate and T cell-mediated intestinal inflammation. The Journal of experimental medicine 203, 2473-2483 (2006).
89. D. Yen, J. Cheung, H. Scheerens, F. Poulet, T. McClanahan, B. McKenzie, M. A. Kleinschek, A. Owyang, J. Mattson, W. Blumenschein, E. Murphy, M. Sathe, D. J. Cua, R. A. Kastelein, D. Rennick, IL-23 is essential for T cell-mediated colitis and promotes inflammation via IL-17 and IL-6. The Journal of clinical investigation 116, 1310-1316 (2006).
90. C. L. Langrish, B. S. McKenzie, N. J. Wilson, R. de Waal Malefyt, R. A. Kastelein, D. J. Cua, IL-12 and IL-23: master regulators of innate and adaptive immunity. Immunological reviews 202, 96-105 (2004).
91. C. Parham, M. Chirica, J. Timans, E. Vaisberg, M. Travis, J. Cheung, S. Pflanz, R. Zhang, K. P. Singh, F. Vega, W. To, J. Wagner, A. M. O'Farrell, T. McClanahan, S. Zurawski, C. Hannum, D. Gorman, D. M. Rennick, R. A. Kastelein, R. de Waal Malefyt, K. W. Moore, A receptor for the heterodimeric cytokine IL-23 is composed of IL-12Rbeta1 and a novel cytokine receptor subunit, IL-23R. J Immunol 168, 5699-5708 (2002).
92. A. T. Stefka, T. Feehley, P. Tripathi, J. Qiu, K. McCoy, S. K. Mazmanian, M. Y. Tjota, G. Y. Seo, S. Cao, B. R. Theriault, D. A. Antonopoulos, L. Zhou, E. B. Chang, Y. X. Fu, C. R. Nagler, Commensal bacteria protect against food allergen sensitization. Proceedings of the National Academy of Sciences of the United States ofAmerica 111, 13145-13150 (2014).
93. M. Derrien, P. Van Baarlen, G. Hooiveld, E. Norin, M. Muller, W. M. de Vos, Modulation of Mucosal Immune Response, Tolerance, and Proliferation in Mice Colonized by the Mucin-Degrader Akkermansia muciniphila. Frontiers in microbiology 2, 166 (2011).
94.A. Everard, C. Belzer, L. Geurts, J. P. Ouwerkerk, C. Druart, L. B. Bindels, Y. Guiot, M. Derrien, G. G. Muccioli, N. M. Delzenne, W. M. de Vos, P. D. Cani, Cross-talk between Akkermansia muciniphila and intestinal epithelium controls diet-induced obesity. Proceedings of the National Academy of Sciences of the United States ofAmerica 110, 9066-9071 (2013).
95. B. P. Ganesh, R. Klopfleisch, G. Loh, M. Blaut, Commensal Akkermansia muciniphila exacerbates gut inflammation in Salmonella Typhimurium infected gnotobiotic mice. PloS one 8, e74963 (2013).
96. T. Pelaseyed, J. H. Bergstrom, J. K. Gustafsson, A. Ermund, G. M. Birchenough, A. Schutte, S. van der Post, F. Svensson, A. M. Rodriguez-Pineiro, E. E. Nystrom, C. Wising, M. E. Johansson, G. C. Hansson, The mucus and mucins of the goblet cells and enterocytes provide the first defense line of the gastrointestinal tract and interact with the immune system. Immunological reviews 260, 8-20 (2014).
97. C. G. Buffie, I. Jarchum, M. Equinda, L. Lipuma, A. Gobourne, A. Viale, C. Ubeda, J. Xavier, E. G. Pamer, Profound alterations of intestinal microbiota following a single dose of clindamycin results in sustained susceptibility to Clostridium difficile-induced colitis. Infection and immunity 80, 62-73 (2012).
98. C. Lozupone, M. Hamady, R. Knight, UniFrac--an online tool for comparing microbial community diversity in a phylogenetic context. BMC bioinformatics 7, 371 (2006).
99.Y. Shono, A. Z. Tuckett, S. Ouk, H. C. Liou, G. Altan-Bonnet, J. J. Tsai, J. E. Oyler, O. M. Smith, M. L. West, N. V. Singer, E. Doubrovina, D. Pankov, C. V. Undhad, G. F. Murphy, C. Lezcano, C. Liu, R. J. O'Reilly, M. R. van den Brink, J. L. Zakrzewski, A small-molecule c-Rel inhibitor reduces alloactivation of T cells without compromising antitumor activity. Cancer discovery 4, 578-591 (2014).
100. K. R. Cooke, L. Kobzik, T. R. Martin, J. Brewer, J. Delmonte, Jr., J. M. Crawford, J. L. Ferrara, An experimental model of idiopathic pneumonia syndrome after bone marrow transplantation: I.The roles of minor H antigens and endotoxin. Blood 88, 3230-3239 (1996).
101. J. L. Zakrzewski, A. A. Kochman, S. X. Lu, T. H. Terwey, T. D. Kim, V. M. Hubbard, S. J. Muriglan, D. Suh, O. M. Smith, J. Grubin, N. Patel, A. Chow, J. Cabrera-Perez, R. Radhakrishnan, A. Diab, M. A. Perales, G. Rizzuto, E. Menet, E. G. Pamer, G. Heller, J. C. Zuniga-Pflucker, O. Alpdogan, M. R. van den Brink, Adoptive transfer of T-cell precursors enhances T-cell reconstitution after allogeneic hematopoietic stem cell transplantation. Nature medicine 12, 1039-1047 (2006).
102. A. M. Bolger, M. Lohse, B. Usadel, Trimmomatic: a flexible trimmer for Illumina sequence data. Bioinformatics 30, 2114-2120 (2014).
103. D. E. Wood, S. L. Salzberg, Kraken: ultrafast metagenomic sequence classification using exact alignments. Genome biology 15, R46 (2014).
104. S. Abubucker, N. Segata, J. Goll, A. M. Schubert, J. Izard, B. L. Cantarel, B. Rodriguez-Mueller, J. Zucker, M. Thiagarajan, B. Henrissat, O. White, S. T. Kelley, B. Methe, P. D. Schloss, D. Gevers, M. Mitreva, C. Huttenhower, Metabolic reconstruction for metagenomic data and its application to the human microbiome. PLoS computational biology 8, e1002358 (2012).
105. M. E. Johansson, J. M. Larsson, G. C. Hansson, The two mucus layers of colon are organized by the MUC2 mucin, whereas the outer layer is a legislator of host-microbial interactions. Proceedings of the National Academy of Sciences of the United States ofAmerica 108 Suppl 1, 4659-4665 (2011).
106. M. Chromek, I. Arvidsson, D. Karpman, The antimicrobial peptide cathelicidin protects mice from Escherichia coli O157:H7-mediated disease. PloS one 7, e46476 (2012).
107. N. H. Salzman, H. de Jong, Y. Paterson, H. J. Harmsen, G. W. Welling, N. A. Bos, Analysis of 16S libraries of mouse gastrointestinal microflora reveals a large new group of mouse intestinal bacteria. Microbiology 148, 3651-3660 (2002).
108. A. Swidsinski, J. Weber, V. Loening-Baucke, L. P. Hale, H. Lochs, Spatial organization and composition of the mucosal flora in patients with inflammatory bowel disease. Journal of clinical microbiology 43, 3380-3389 (2005).
109. D. M. Jacobs, N. Deltimple, E. van Velzen, F. A. van Dorsten, M. Bingham, E. E. Vaughan, J. van Duynhoven, (1)H NMR metabolite profiling of feces as a tool to assess the impact of nutrition on the human microbiome. NMR in biomedicine 21, 615-626 (2008).
110. Y. S. Hong, Y. T. Ahn, J. C. Park, J. H. Lee, H. Lee, C. S. Huh, D. H. Kim, H. Ryu do,
   G. S. Hwang, 1H NMR-based metabonomic assessment of probiotic effects in a colitis mouse model. Archives of pharmacal research 33, 1091-1101 (2010).

## Claims

1. A therapeutic composition for use in treating a graft versus host disease (GVHD) in a subject following bone marrow transplant (BMT) or hematopoietic stem cell transplant (HSCT), wherein the therapeutic composition comprises one or more purified populations of bacteria selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* and combinations or mixtures thereof.

2. The therapeutic composition for use of claim 1, wherein said bacteria comprise a 16S rDNA with the nucleotide sequence of one of SEQ ID NOS: 1, 3, 4, 5, 7, 8, 9, 12, and 15 or a nucleotide sequence with about 98% to 100% identity to said sequence.

3. The therapeutic composition for use of claim 1 or 2,
wherein the bacteria in said composition are live bacteria, frozen bacteria, germinatable spores, or a combination thereof; and/or
wherein the bacteria ferment an oligosaccharide selected from xylose, raffinose, cellobiose, or melizitose.

4. The therapeutic composition for use of any one of claims 1-3,
wherein the bacteria are present in a dose of 10⁴ to 10¹⁰ CFUs; or
wherein the bacteria are present in a dose of 10⁵ to 10⁹ CFUs; or
wherein the bacteria are present in a dose of 10⁶ to 10⁸ CFUs.

5. The therapeutic composition for use of any one of claims 1-4 formulated for oral administration; or formulated for colonic/rectal administration.

6. A therapeutic composition for use in preventing, reducing the likelihood of, and/or treating a graft versus host disease (GVHD) in a subject undergoing bone marrow or hematopoietic stem cell transplant, wherein the therapeutic composition comprises one or more purified populations of bacteria selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* and combinations or mixtures thereof.

7. The therapeutic composition for use of claim 6, wherein the composition
(i) stimulates the growth or activity of one or more bacterial taxa which are underrepresented in microbiota of the subject either before transplant or following transplant; or
(ii) inhibits the growth or activity of one or more bacterial taxa which are over-represented in microbiota of the subject.

8. The therapeutic composition for use of claim 6 or 7,
wherein the bone marrow transplant is allogeneic bone marrow transplant (allo-BMT); and/or
wherein the hematopoietic stem cell transplant is allogeneic hematopoietic stem cell transplant (allo-HSCT).

9. The therapeutic composition for use of claim 8,
wherein the composition is to be administered to the subject from about 1 day to about 2 weeks following cessation of a treatment with antibiotics with high activity against anaerobes; or
wherein said composition is to be administered to the subject from about 7 days to about 10 days before allo-BMT or allo-HSCT; or
wherein said composition is to be administered to the subject from about 1 day to about 1 week before allo-BMT or allo-HSCT.

10. A method for screening a subject for risk of developing GVHD following bone marrow transplant (BMT) or hematopoietic stem cell transplant (HSCT), the method comprising determining the abundance of a bacterial species of the order *Clostridiales* in a sample of fecal material from the subject, wherein the subject is at an increased risk for GVHD if the abundance of said *Clostridiales* in said sample of fecal material is less than the abundance of said *Clostridiales* in a sample of fecal material from a subject who is not at risk for developing GVHD, and wherein said *Clostridiales* species is selected from the group consisting of *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli* and combinations or mixtures thereof.

11. The method of claim 10,
wherein the abundance of the *Clostridiales* species is less than from 0.5% to 0.01%; or
wherein the abundance of the *Clostridiales* species is less than from 0.25% to 0.02%; or
wherein the abundance of the *Clostridiales* species is less than 0.05%; or
wherein said *Clostridiales* comprise a 16S rDNA with the nucleotide sequence of one of SEQ ID NOS: 1-16 or a nucleotide sequence with about 98% to 100% identity to said sequence.

## Patentansprüche

1. Therapeutische Zusammensetzung zur Verwendung bei dem Behandeln einer Transplantat-gegen-Wirt-Reaktion (GVHD) bei einem Subjekt nach einer Knochenmarktransplantation (BMT) oder einer hämatopoetischen Stammzellentransplantation (HSCT), wobei die therapeutische Zusammensetzung eine oder mehrere gereinigte Populationen von Bakterien umfasst, ausgewählt aus der Gruppe, bestehend aus *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* und Kombinationen oder Mischungen davon.

2. Therapeutische Zusammensetzung zur Verwendung nach Anspruch 1, wobei die Bakterien eine 16S rDNA mit der Nukleotidsequenz einer der SEQ ID NOS: 1, 3, 4, 5, 7, 8, 9, 12 und 15 oder einer Nukleotidsequenz mit etwa 98 % bis 100 % Identität mit der Sequenz umfassen.

3. Therapeutische Zusammensetzung zur Verwendung nach Anspruch 1 oder 2,
wobei die Bakterien in der Zusammensetzung lebende Bakterien, gefrorene Bakterien, keimfähige Sporen oder eine Kombination davon sind; und/oder
wobei die Bakterien ein Oligosaccharid, ausgewählt aus Xylose, Raffinose, Cellobiose oder Melizitose, fermentieren.

4. Therapeutische Zusammensetzung zur Verwendung nach einem der Ansprüche 1 bis 3,
wobei die Bakterien in einer Dosis von 10⁴ bis 10¹⁰ KBEs anwesend sind; oder
wobei die Bakterien in einer Dosis von 10⁵ bis 10⁹ KBEs anwesend sind; oder
wobei die Bakterien in einer Dosis von 10⁶ bis 10⁸ KBEs anwesend sind.

5. Therapeutische Zusammensetzung zur Verwendung nach einem der Ansprüche 1 bis 4, formuliert zur oralen Verabreichung; oder formuliert zur kolonalen/rektalen Verabreichung.

6. Therapeutische Zusammensetzung zur Verwendung bei dem Vorbeugen, dem Reduzieren der Wahrscheinlichkeit und/oder dem Behandeln einer Transplantat-gegen-Wirt-Reaktion (GVHD) bei einem Subjekt, an dem eine Knochenmark- oder hämatopoetische Stammzellentransplantation vorgenommen wird, wobei die therapeutische Zusammensetzung eine oder mehrere gereinigte Populationen von Bakterien umfasst, ausgewählt aus der Gruppe, bestehend aus *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* und Kombinationen oder Mischungen davon.

7. Therapeutische Zusammensetzung zur Verwendung nach Anspruch 6, wobei die Zusammensetzung
(i) das Wachstum oder die Aktivität eines oder mehrerer Bakterientaxa stimuliert, die in der Mikrobiota des Subjekts entweder vor der Transplantation oder nach der Transplantation unterrepräsentiert sind; oder
(ii) das Wachstum oder die Aktivität eines oder mehrerer Bakterientaxa hemmt, die in der Mikrobiota des Subjekts überrepräsentiert sind.

8. Therapeutische Zusammensetzung zur Verwendung nach Anspruch 6 oder 7,
wobei die Knochenmarktransplantation eine allogene Knochenmarktransplantation (allo-BMT) ist; und/oder
wobei die hämatopoetische Stammzellentransplantation eine allogene hämatopoetische Stammzellentransplantation (allo-HSCT) ist.

9. Therapeutische Zusammensetzung zur Verwendung nach Anspruch 8,
wobei die Zusammensetzung dem Subjekt etwa 1 Tag bis etwa 2 Wochen nach Beendigung einer Behandlung mit Antibiotika mit hoher Aktivität gegen Anaerobier zu verabreichen ist; oder
wobei die Zusammensetzung dem Subjekt etwa 7 Tage bis etwa 10 Tage vor der allo-BMT oder allo-HSCT zu verabreichen ist; oder
wobei die Zusammensetzung dem Subjekt etwa 1 Tag bis etwa 1 Woche vor der allo-BMT oder allo-HSCT zu verabreichen ist.

10. Verfahren zum Screening eines Subjekts auf das Risiko, nach einer Knochenmarktransplantation (BMT) oder einer hämatopoetischen Stammzellentransplantation (HSCT) eine GVHD zu entwickeln, das Verfahren umfassend das Bestimmen der Häufigkeit einer bakteriellen Spezies der Ordnung *Clostridiales* in einer Probe von fäkalem Material des Subjekts, wobei das Subjekt ein erhöhtes Risiko für GVHD hat, wenn die Häufigkeit der *Clostridiales* in der Probe des fäkalen Materials geringer ist als die Häufigkeit der *Clostridiales* in einer Probe des fäkalen Materials eines Subjekts, das kein Risiko hat, GVHD zu entwickeln, und wobei die *Clostridiales-Spezies* aus der Gruppe, bestehend aus *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli* und Kombinationen oder Mischungen davon, ausgewählt ist.

11. Verfahren nach Anspruch 10,
wobei die Häufigkeit der *Clostridiales-Spezies* weniger als von 0,5 % bis 0,01 % ist; oder
wobei die Häufigkeit der *Clostridiales-Spezies* weniger als von 0,25 % bis 0,02 % ist; oder
wobei die Häufigkeit der *Clostridiales-Spezies* weniger als 0,05 % ist; oder
wobei die *Clostridiales* eine 16S rDNA mit der Nukleotidsequenz einer der SEQ ID NOS: 1-16 oder einer Nukleotidsequenz mit etwa 98 % bis 100 % Identität mit der Sequenz umfassen.

## Revendications

1. Composition thérapeutique destinée à être utilisée dans le traitement d'une maladie du greffon contre l'hôte (GVHD) chez un sujet après une greffe de moelle osseuse (BMT) ou une greffe de cellules souches hématopoïétiques (HSCT), dans laquelle la composition thérapeutique comprend une ou plusieurs populations purifiées de bactéries choisies dans le groupe constitué par *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* et des combinaisons ou mélanges de celles-ci.

2. Composition thérapeutique destinée à être utilisée selon la revendication 1, dans laquelle lesdites bactéries comprennent un ADNr 16S avec la séquence nucléotidique de l'une des SEQ ID NO : 1, 3, 4, 5, 7, 8, 9, 12 et 15 ou une séquence nucléotidique avec environ 98 % à 100 % d'identité avec ladite séquence.

3. Composition thérapeutique destinée à être utilisée selon la revendication 1 ou 2,
dans laquelle les bactéries dans ladite composition sont des bactéries vivantes, des bactéries congelées, des spores germinables ou une combinaison de celles-ci ; et/ou
dans laquelle les bactéries fermentent un oligosaccharide choisi parmi le xylose, le raffinose, le cellobiose ou le mélizitose.

4. Composition thérapeutique destinée à être utilisée selon l'une quelconque des revendications 1 à 3,
dans laquelle les bactéries sont présentes à un dose de 10⁴ à 10¹⁰ UFC ; ou
dans laquelle les bactéries sont présentes à un dose de 10⁵ à 10⁹ UFC ; ou
dans laquelle les bactéries sont présentes à un dose de 10⁶ à 10⁸ UFC ; ou

5. Composition thérapeutique destinée à être utilisée selon l'une quelconque des revendications 1 à 4, formulée pour une administration orale ; ou formulée pour une administration colonique/rectale.

6. Composition thérapeutique destinée à être utilisée dans la prévention, la réduction de la probabilité de et/ou le traitement d'une maladie du greffon contre l'hôte (GVHD) chez un sujet après une greffe de moelle osseuse ou de cellules souches hématopoïétiques, dans laquelle la composition thérapeutique comprenant une ou plusieurs populations purifiées de bactéries choisies dans le groupe constitué par *Ruminococcus obeum, Clostridium hathewayi, Eubacterium desmolans, Dorea longicatena, Ruminococcus lactaris (Blautia producta), Eubacterium contortum, Ruminococcus faecis, Holdemania filiformis, Clostridium sordelli,* et des combinaisons ou mélanges de celles-ci.

7. Composition thérapeutique destinée à être utilisée selon la revendication 6, dans laquelle la composition
(i) stimule la croissance ou l'activité d'un ou plusieurs taxons bactériens qui sont sous-représentés dans le microbiote du sujet soit avant la transplantation, soit après la transplantation ; ou
(ii) inhibe la croissance ou l'activité d'un ou plusieurs taxons bactériens sur-représentés dans le microbiote du sujet.

8. Composition thérapeutique destinée à être utilisée selon la revendication 6 ou 7,
dans laquelle la greffe de moelle osseuse est une greffe de moelle osseuse allogénique (allo-BMT) ; et/ou
dans laquelle la greffe de cellules souches hématopoïétiques est une greffe allogénique de cellules souches hématopoïétiques (allo-HSCT).

9. Composition thérapeutique destinée à être utilisée selon la revendication 8,
dans laquelle la composition doit être administrée au sujet environ 1 jour à environ 2 semaines après l'arrêt d'un traitement avec des antibiotiques à forte activité contre les anaérobies ; ou
dans laquelle ladite composition doit être administrée au sujet environ 7 jours à environ 10 jours avant l'allo-BMT ou l'allo-HSCT ; ou
dans laquelle ladite composition doit être administrée au sujet environ 1 jour à environ 1 semaine avant l'allo-BMT ou l'allo-HSCT.

10. Procédé de dépistage d'un risque de développer une GVHD chez un sujet après une greffe de moelle osseuse (BMT) ou une greffe de cellules souches hématopoïétiques (HSCT), le procédé comprenant la détermination de l'abondance d'une espèce bactérienne de l'ordre des *Clostridiales* dans un échantillon de matière fécale du sujet, dans lequel le sujet présente un risque accru de GVHD si l'abondance desdites *Clostridiales* dans ledit échantillon de matière fécale est inférieure à l'abondance desdites *Clostridiales* dans un échantillon de matière fécale d'un sujet qui ne présente pas de risque de développer une GVHD, et dans lequel ladite espèce de *Clostridiales* est choisie dans le groupe constitué par *Ruminococcus obeum, Clostridium hathewayi, Eubacterium desmolans, Dores longicatena, Ruminococcus lactaris (Blautia producta), Eubacterium contortum, Ruminococcus faecis, Holdemania filiformis, Clostridium sordelli* et des combinaisons ou mélanges de celles-ci.

11. Procédé selon la revendication 10,
dans lequel l'abondance des espèces *Clostridiales* est inférieure à la plage comprise entre 0,5 % et 0,01 % ; ou
dans lequel l'abondance des espèces *Clostridiales* est inférieure à la plage comprise entre 0,25 % et 0,02 % ; ou
dans lequel l'abondance des espèces *Clostridiales* est inférieure à 0,05 % ; ou
dans lequel lesdits *Clostridiales* comprennent un ADNr 16S avec la séquence nucléotidique de l'une des SEQ ID NO : 1 à 16 ou une séquence nucléotidique avec environ 98 % à 100 % d'identité avec ladite séquence.
